# EUROPEAN PATENT APPLICATION

(11) **EP 4 488 382 A2**
(43) Date of publication of application: **08.01.2025**
(21) Application number: 24208929.0
(22) Date of filing: 01.08.2018
(51) Int. Cl.: C12Q 1/6806

(54) **HYDROGEL BEADS FOR NUCLEOTIDE SEQUENCING**

(30) Priority: 01.08.2017 US 201762539956 P
(62) Divisional of application: 18755643.6
(71) Applicant: Illumina, Inc., San Diego, CA 92122 (US)
(72) Inventor: WU, Yir-Shyuan, San Diego, CA 92122 (US); KHURANA, Tarun Kumar, San Diego, CA 92122 (US); MASHAYEKHI, Foad, San Diego, CA 92122 (US); DAGGUMATI, Pallavi, San Diego, CA 92122 (US); STEEMERS, Frank J., San Diego, CA 92122 (US); GUNDERSON, Kevin L., San Diego, CA 92122 (US); FISHER, Jeffrey S., San Diego, CA 92122 (US); CHEN, Xi-Jun, San Diego, CA 92122 (US); GORPE-YASAR, Filiz, San Diego, CA 92122 (US); NOÉ, Falko, San Diego, CA 92122 (US); LIN, Charles, San Diego, CA 92122 (US)
(74) Representative: Murgitroyd & Company

(57) **Abstract**

Systems, methods, and compositions provided herein relate to preparation of beads encapsulating genetic material. Some embodiments include preparation of nucleic acid libraries within the bead, wherein the bead includes pores that allow diffusion of reagents while retaining genetic material.

## Description

### CROSS-REFERENCE TO RELATED APPLICATIONS

This application claims priority to U.S. Prov. App. No. 62/539,956 filed August 1, 2017 entitled "HYDROGEL BEADS FOR NUCLEOTIDE SEQUENCING" which is incorporated by reference herein in its entirety.

### FIELD

Systems, methods, and compositions provided herein relate to hydrogel beads, and methods of encapsulating material within hydrogel beads, for use in spatial index sequencing and nucleic acid library preparation.

### BACKGROUND

Next generation sequencers are powerful tools that generate large amounts of genomic data per sequencing run. Interpreting and analyzing this large amount of data can be challenging. Single cell DNA sequencing is emerging as one tool for studying genomic heterogeneity. Specifically, the microbiome, which carries multiple repeated genomic regions, can be sequenced by obtaining DNA sequences from only a single cell. This will largely simplify the downstream sequencing data alignment process.

### SUMMARY

Some embodiments provided herein relate to a bead for performing nucleic acid reactions. In some embodiments, the bead includes a hydrogel polymer and genetic material disposed within the hydrogel polymer. In some embodiments, the bead includes pores that allow diffusion of a reagent through the bead while retaining the genetic material. In some embodiments, the bead has a diameter of about 2 µm to about 120 µm, such as, for example, 2, 3, 4, 5, 6, 7, 8, 9, 10, 15, 20, 25, 30, 35, 40, 45, 50, 60, 70, 80, 90, 100, 110, or 120 µm in diameter, or a diameter within a range defined by any two of the aforementioned values. In some embodiments, the hydrogel polymer includes polyethylene glycol (PEG)-thiol, PEG-acrylate, acrylamide, N,N'-bis(acryloyl)cystamine (BACy), PEG, polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, collagen, bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations thereof. In some embodiments, the hydrogel polymer includes polyethylene glycol (PEG)-thiol/PEG-acrylate. In some embodiments, the hydrogel polymer includes acrylamide/N,N'-bis(acryloyl)cystamine (BACy). In some embodiments, the hydrogel polymer includes PEG/polypropylene oxide (PPO). In some embodiments, the genetic material is a cell, a nucleic acid, or a microbiome. In some embodiments, the cell is a mammalian cell or bacterial cell. In some embodiments, the cell is an *Escherichia coli* cell, a *Bacillus subtilis* cell, an *Aeromonas hydrophila* cell, or a fibroblast cell. In some embodiments, the nucleic acid is DNA or RNA of 300 base pairs or greater. In some embodiments, the reagent includes enzymes, chemicals, and primers having a size of less than 50 base pairs. In some embodiments, the reagent includes lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations.

Some embodiments provided herein relate to a method of encapsulating genetic material within a hydrogel bead. In some embodiments, the method includes mixing genetic material with a hydrogel polymer in solution and mixing the solution with an immiscible fluid to form a hydrogel bead encapsulating the genetic material. In some embodiments, the hydrogel bead includes pores that allow diffusion of a reagent through the hydrogel bead, while retaining the genetic material. In some embodiments, the hydrogel bead has a diameter of about 2 µm to about 120 µm, such as, for example, 2, 3, 4, 5, 6, 7, 8, 9, 10, 15, 20, 25, 30, 35, 40, 45, 50, 60, 70, 80, 90, 100, 110, or 120 µm in diameter, or a diameter within a range defined by any two of the aforementioned values. In some embodiments, the hydrogel polymer includes polyethylene glycol (PEG)-thiol, PEG-acrylate, acrylamide, N,N'-bis(acryloyl)cystamine (BACy), PEG, polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, collagen, bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations thereof. In some embodiments, the hydrogel polymer includes polyethylene glycol (PEG)-thiol/PEG-acrylate. In some embodiments, the hydrogel polymer includes acrylamide/N,N'-bis(acryloyl)cystamine (BACy). In some embodiments, the hydrogel polymer includes PEG/polypropylene oxide (PPO). In some embodiments, the genetic material is a cell, a nucleic acid, or a microbiome. In some embodiments, the cell is a mammalian cell or bacterial cell. In some embodiments, the cell is an *Escherichia coli* cell, a *Bacillus subtilis* cell, an *Aeromonas hydrophila* cell, or a fibroblast cell. In some embodiments, the nucleic acid is DNA or RNA of 300 base pairs or greater. In some embodiments, the reagent includes enzymes, chemicals, and primers having a size of less than 50 base pairs. In some embodiments, the reagent comprises lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations.

Some embodiments provided herein relate to a method of encapsulating genetic material within a hydrogel bead. In some embodiments, the method includes mixing an aqueous solution including a hydrogel polymer and genetic material with an immiscible fluid, inputting the aqueous solution into a droplet generator, and generating a hydrogel bead encapsulating the genetic material. In some embodiments, the hydrogel bead includes pores that allow diffusion of a reagent through the hydrogel bead while retaining the genetic material. In some embodiments, the hydrogel bead has a diameter of about 2 µm to about 120 µm, such as, for example, 2, 3, 4, 5, 6, 7, 8, 9, 10, 15, 20, 25, 30, 35, 40, 45, 50, 60, 70, 80, 90, 100, 110, or 120 µm in diameter, or a diameter within a range defined by any two of the aforementioned values. In some embodiments, the hydrogel polymer includes polyethylene glycol (PEG)-thiol, PEG-acrylate, acrylamide, N,N'-bis(acryloyl)cystamine (BACy), PEG, polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, collagen, bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations thereof. In some embodiments, the hydrogel polymer includes polyethylene glycol (PEG)-thiol/PEG-acrylate. In some embodiments, the hydrogel polymer includes acrylamide/N,N'-bis(acryloyl)cystamine (BACy). In some embodiments, the hydrogel polymer includes PEG/polypropylene oxide (PPO). In some embodiments, the genetic material is a cell, a nucleic acid, or a microbiome. In some embodiments, the cell is a mammalian cell or bacterial cell. In some embodiments, the cell is an *Escherichia coli* cell, a *Bacillus subtilis* cell, an *Aeromonas hydrophila* cell, or a fibroblast cell. In some embodiments, the nucleic acid is DNA or RNA of 300 base pairs or greater. In some embodiments, the reagent includes enzymes, chemicals, and primers having a size of less than 50 base pairs. In some embodiments, the reagent includes lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations.

Some embodiments provided herein relate to a method of preparing a nucleic acid library from genetic material encapsulated within a bead. In some embodiments the method includes obtaining a bead having genetic material disposed therein, as described herein, amplifying genetic material encapsulated within the bead, performing a tagmentation reaction on the genetic material encapsulated within the bead, and sequencing the genetic material, thereby generating a nucleic acid library encapsulated within the bead. In some embodiments, the genetic material is a cell, and the method further includes lysing the cell and extracting nucleic acids. In some embodiments, the cell is lysed with lysozyme and treated with proteinase K to extract nucleic acids. In some embodiments, the tagmentation reaction includes contacting genetic material with a transposase mixture including adapter sequences and transposomes. In some embodiments, the method further includes seeding the nucleic acid library on a solid support. In some embodiments, seeding includes degrading the bead to release the nucleic acid library from the bead. In some embodiments, the bead is degraded by contacting the bead with a cleavage mix or by heating the bead to about 90°C to release the nucleic acid library. In some embodiments, the cleavage mix includes (DTT), tris(2-carboxyethyl)phosphine (TCEP), or tris(3-hydroxypropyl)phosphine (THP). In some embodiments, the solid support is a flow cell device.

### BRIEF DESCRIPTION OF THE DRAWINGS

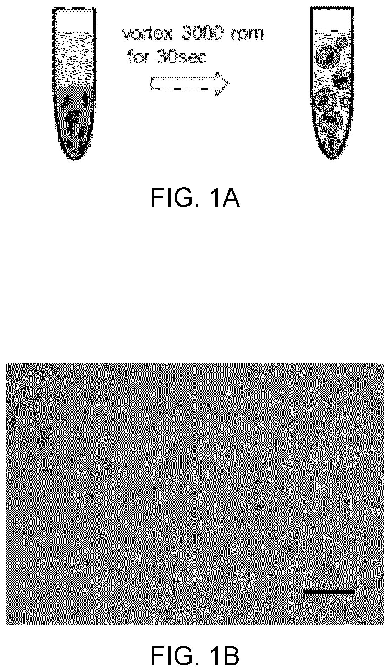

FIG. 1A is a schematic that illustrates an embodiment of preparing hydrogel beads by manual vortexing. FIG. 1B is a micrograph image showing beads formed by this process, wherein the beads were found to range from about 2 µm to about 100 µm in diameter.

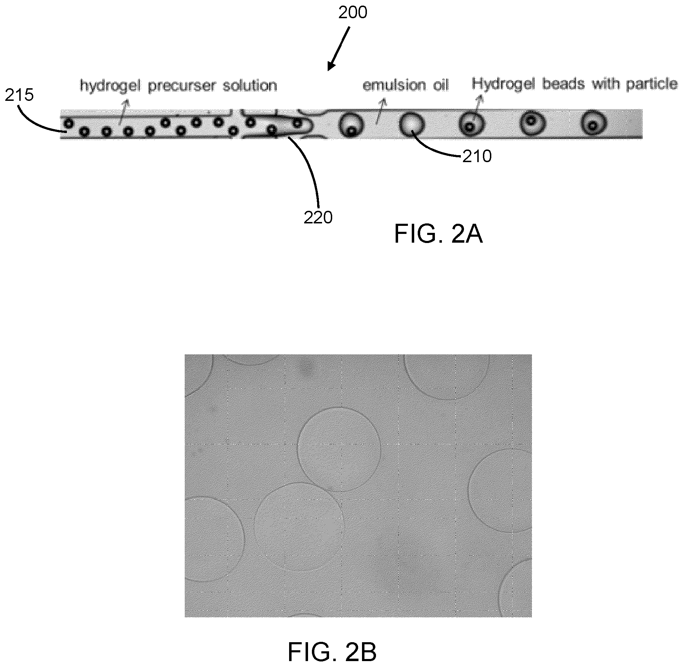

FIG. 2A is a schematic that illustrates an embodiment for preparing hydrogel beads using a microfluidic droplet generator. FIG. 2B is a micrograph image showing beads formed by this process, wherein the beads are of uniform size based on the dimensions of the microfluidic droplet generator.

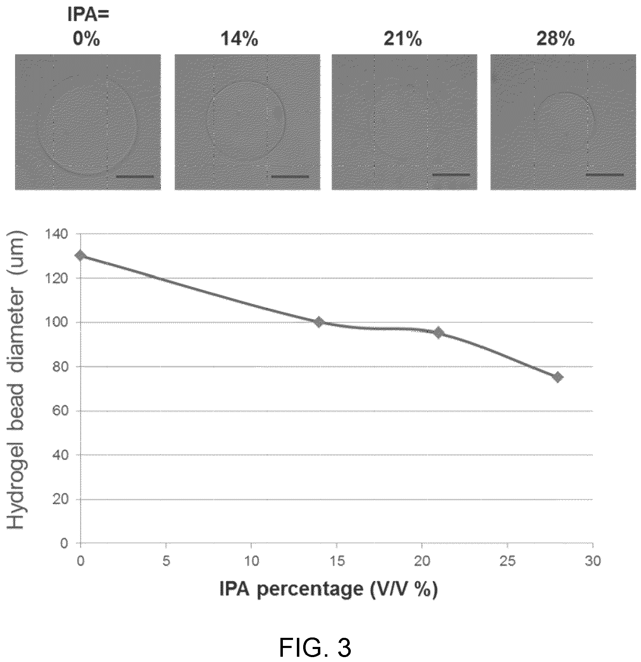

FIG. 3 includes a line chart illustrating the effect of adding various concentrations of isopropyl alcohol to hydrogel polymers using the same microfluidic droplet generation as described in FIG. 2. FIG. 3 also shows several micrographs of hydrogel polymers of differing sizes.

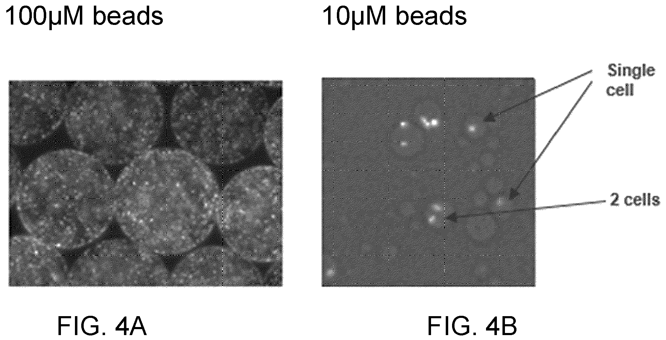

FIGs. 4A and 4B are micrographs of fluorescent dye stained bacterial cells trapped in different size hydrogels. FIG 4A depicts hydrogel beads of about 100 µm diameter encapsulating numerous bacterial cells. FIG. 4B depicts hydrogel beads of about 10 µm dimeter encapsulating one or two bacterial cells.

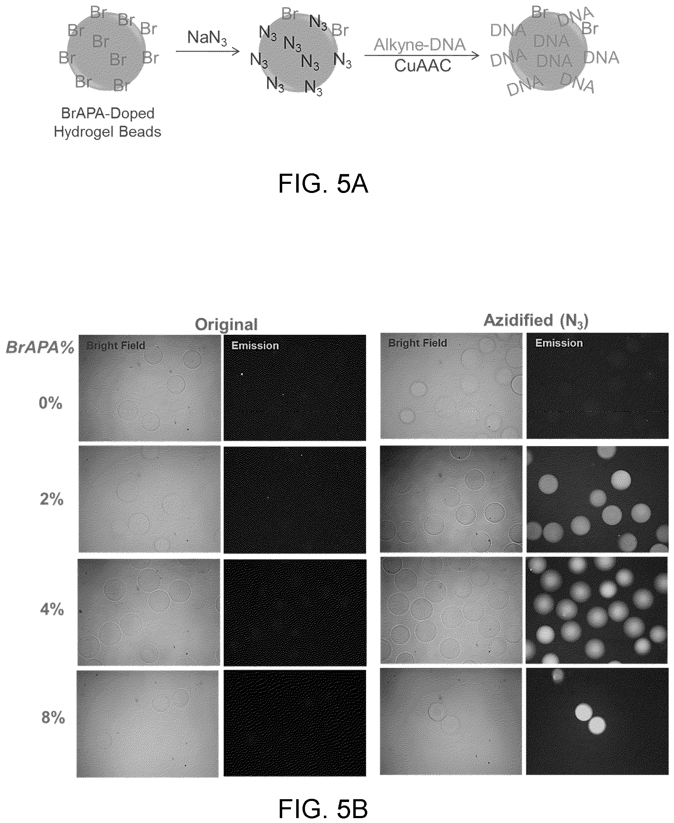

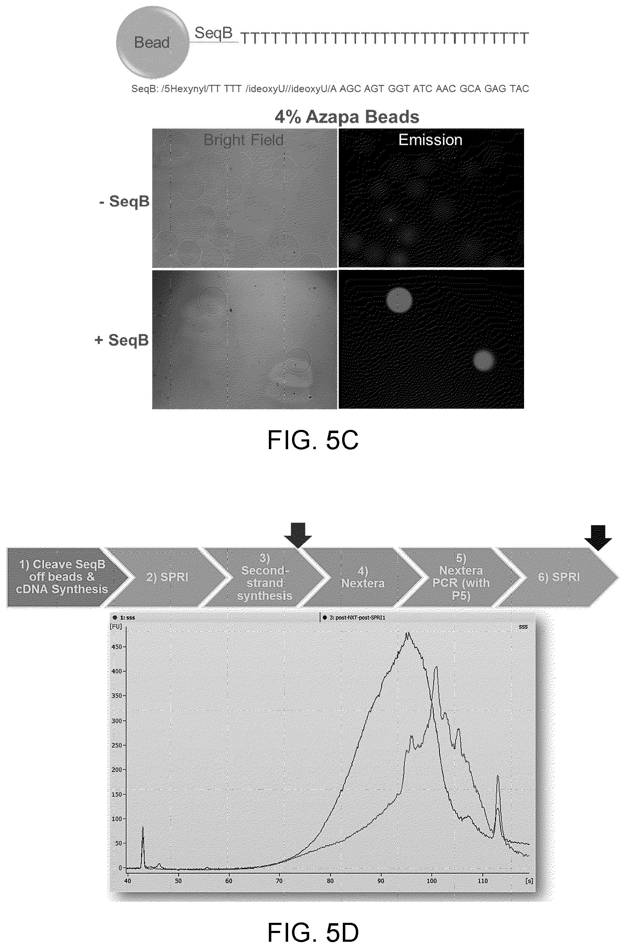

FIG. 5A illustrates an embodiment for functionalizing hydrogel beads with oligonucleotides. FIG. 5B shows micrographs of oligonucleotide functionalized hydrogel beads with and without azidification. FIG. 5C shows micrographs of polyT functionalized hydrogel beads capturing a SeqB sequence. FIG. 5D shows an electrochromatogram of DNA following second strand synthesis (right curve) and following tagmentation (left curve).

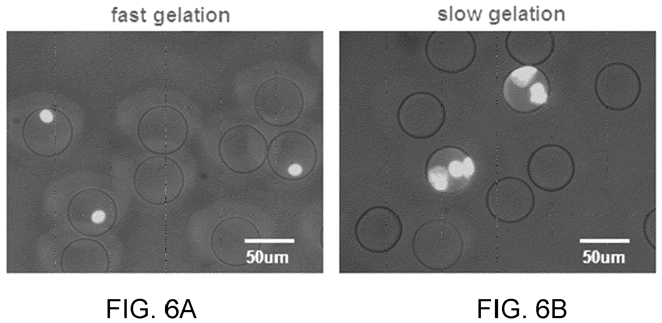

FIGs. 6A and 6B are micrographs showing mouse fibroblast cells (NIH-3T3) encapsulated within hydrogel beads. FIG. 6A shows that a single cell is encapsulated under fast gelation conditions. FIG. 6B shows that multiple cells are encapsulated by the hydrogel under slow gelation conditions.

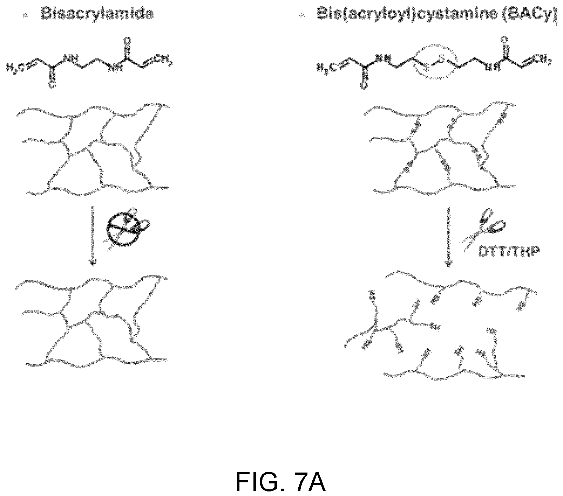

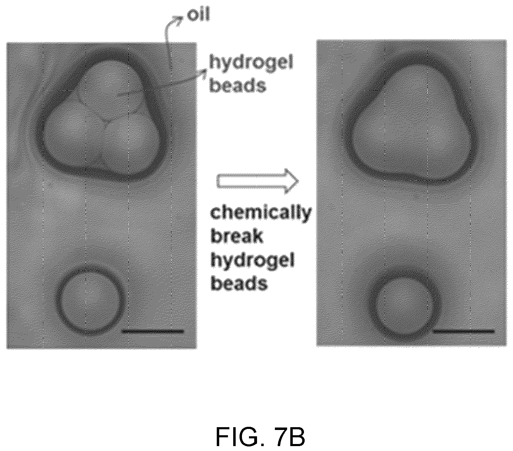

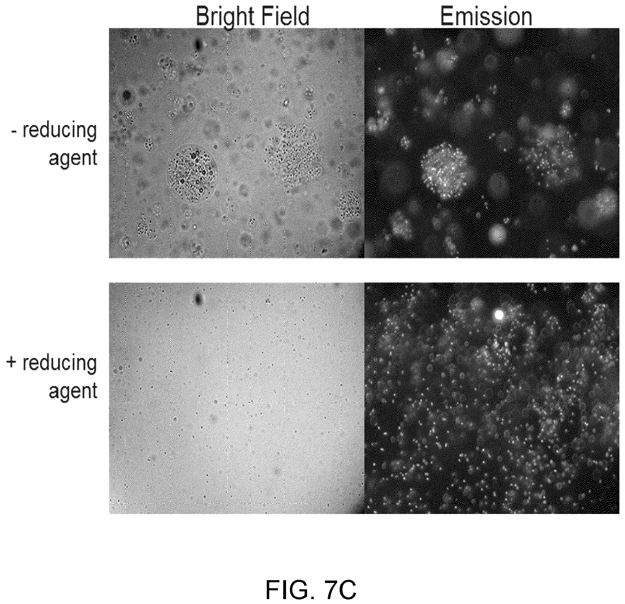

FIG. 7A illustrates an embodiment of the process of making a hydrogel polymer having disulfide bonds, which are cleavable in the presence of a reducing agent, such as dithiothreitol (DTT), tris(2-carboxyethyl)phosphine (TCEP), or tris(3-hydroxypropyl)phosphine (THP). FIG. 7B illustrates micrographs of hydrogel beads that have been chemically cleaved, allowing release of the encapsulated genetic material. FIG. 7C are micrographs that illustrate the release of encapsulated genetic material from hydrogel beads.

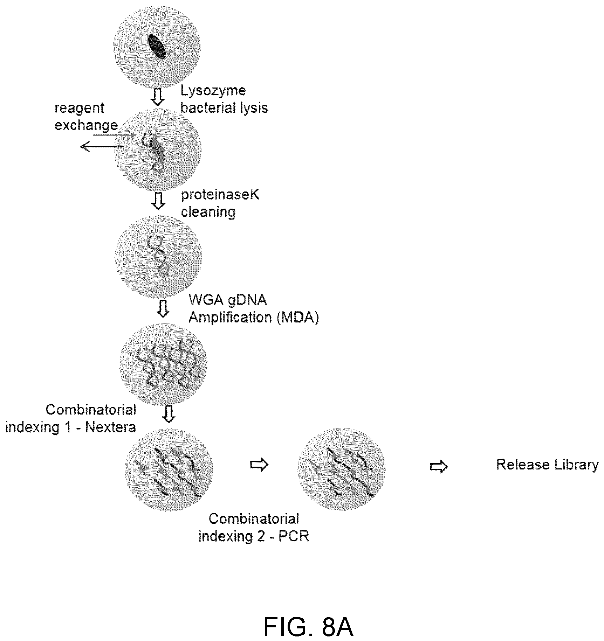

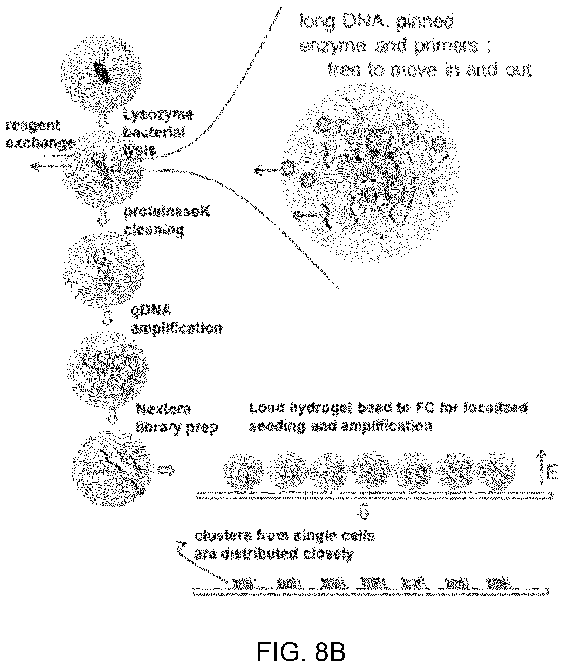

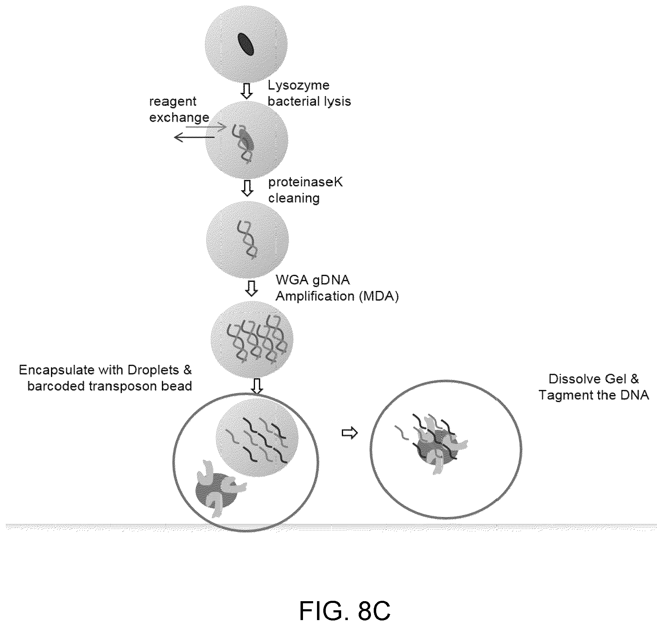

FIGs. 8A-8C illustrate steps for one embodiment of using encapsulated genetic material for in-gel barcoding using combinatorial indexing (FIG. 8A), sequencing of single bacterial cells encapsulated within hydrogel beads (FIG. 8B), and in-gel barcoding using beads containing barcoded transposome complex assembled onto it or barcoded oligonucleotides inserted into tagmented DNA via polymerase chain reaction (PCR) (FIG. 8C).

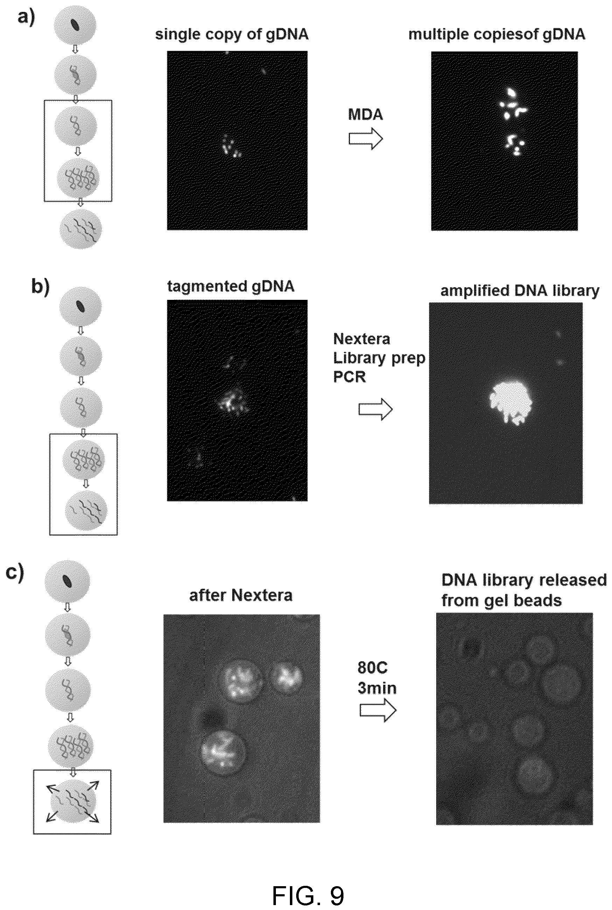

FIG. 9 illustrates steps for DNA amplification within hydrogel beads. Bacterial cells are encapsulated within a hydrogel bead. The captured cells are lysed with lysozyme, and proteins denatured using proteinase K. gDNA In panel a) the gDNA within the encapsulated beads is amplified using multiple displacement amplification (MDA). In panel b), the DNA is tagmented and amplified by PCR. In panel c), the tagmented DNA is eluted out of the hydrogel beads by heating the hydrogel beads to 90°C. Images show fluorescent intensity of DNA stained with SYTOX intercalator dye.

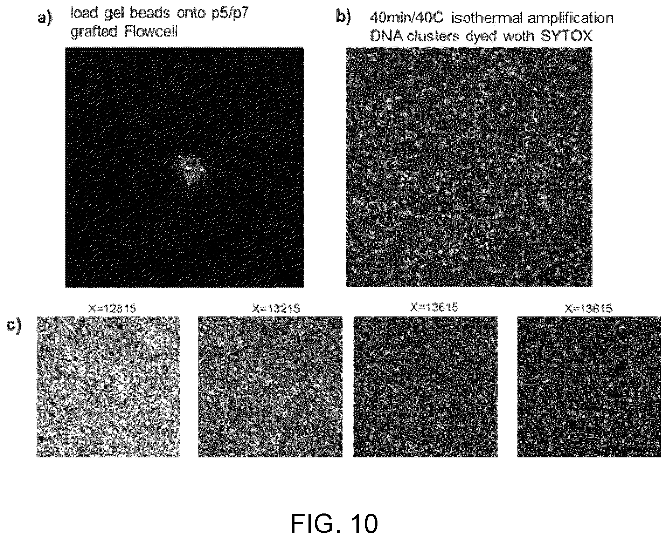

FIG. 10 depicts micrographs that illustrate release of a DNA library on a solid surface for seeding and clustering. Panel a) depicts a single hydrogel bead with tagmented bacterial genome, which is loaded on a solid surface having P5 and P7 primers immobilized thereon. Panel b) shows single stranded DNA that was eluted, seeded, and isothermally bridge amplified. In panel c), a gradient cluster density of seeded amplified DNA is shown. DNA is stained with SYTOX intercalator dye.

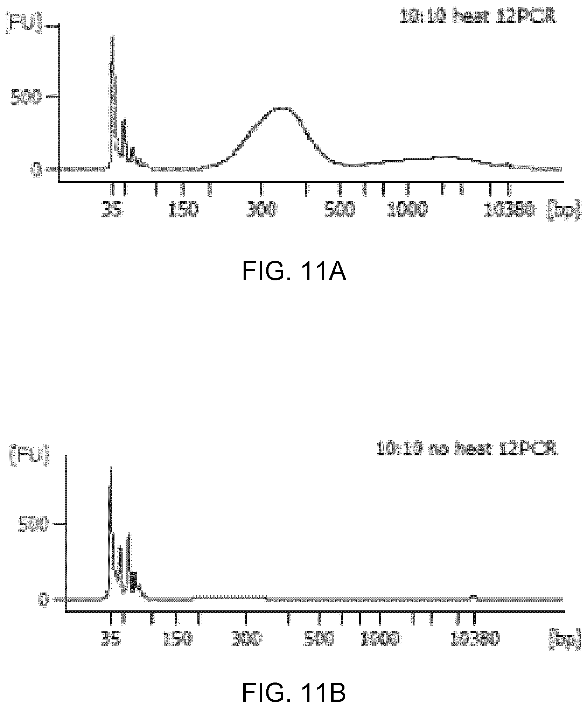

FIG. 11A is a line graph depicting the bioanalyzer trace of a DNA library obtained from 12 cycle PCR of supernatant containing genetic material released from hydrogel beads by heating to 90°C. In FIG. 11B, the hydrogel beads were not heated, and the DNA library is therefore not released from the hydrogel beads, and no library is seen.

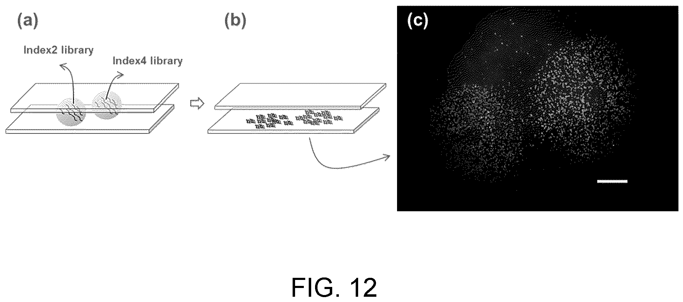

FIG. 12 depict the spatial confinement of DNA sequencing clusters. Panel (a) shows a first-base sequencing readout of two uniquely indexed DNA libraries originating from two distinct hydrogel beads. Panel (b) illustrates the spatial separation of the two libraries. As shown in the micrograph image of panel (c) the separated libraries are spatial distinct and originated from different hydrogel beads.

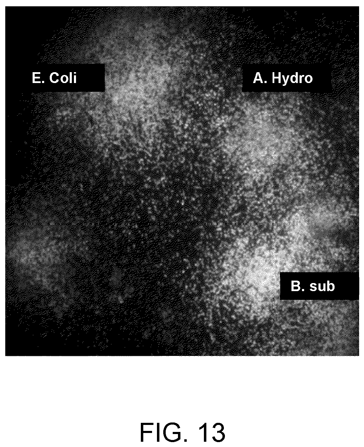

FIG. 13 depicts a micrograph of first base sequencing data of the barcoded hydrogel bead library from three different bacterial cells (*B. subtilis, E. coli,* and *A*. *hydrophila*) with spatial separation.

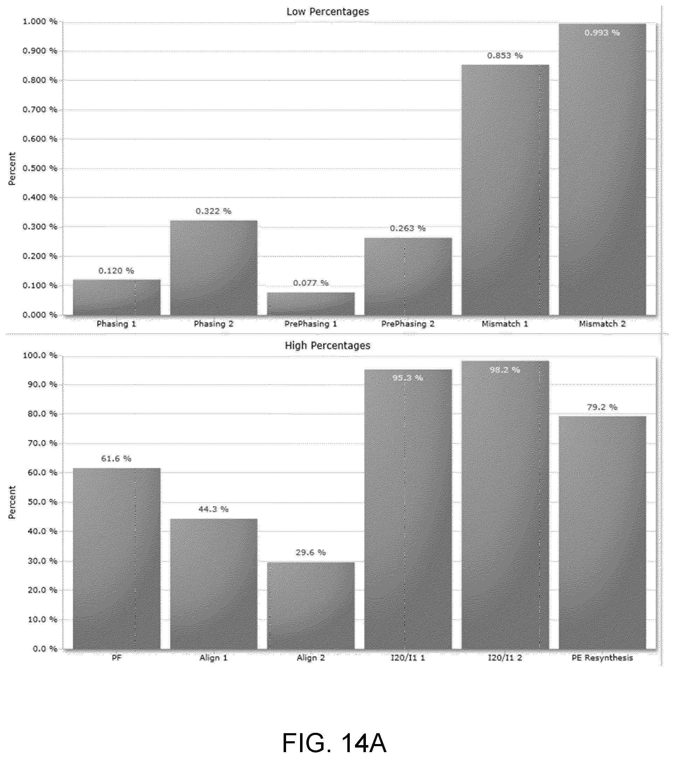

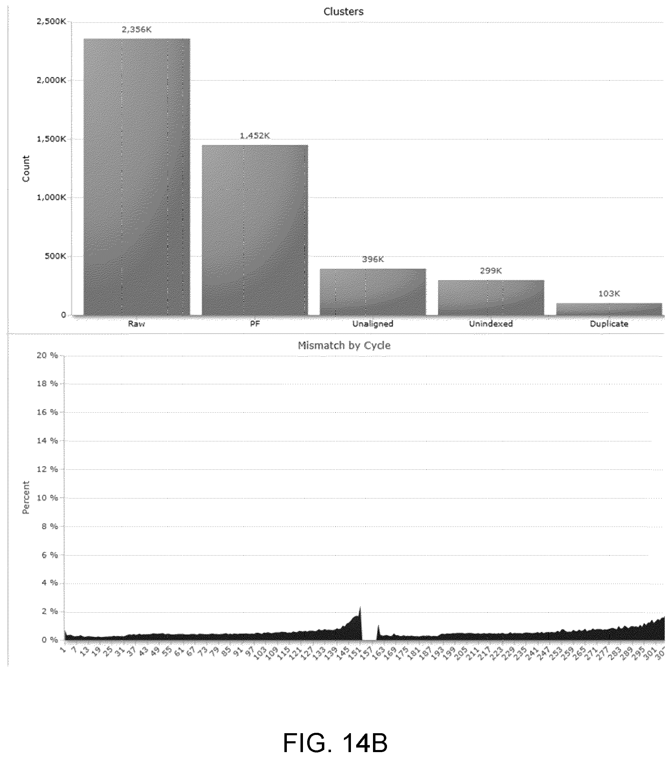

FIGs. 14A-14B depict the sequencing metrics for spatial *indexed B. subtilis, E. coli,* and *A. hydrophila* libraries released from hydrogel beads onto a flow cell surface.

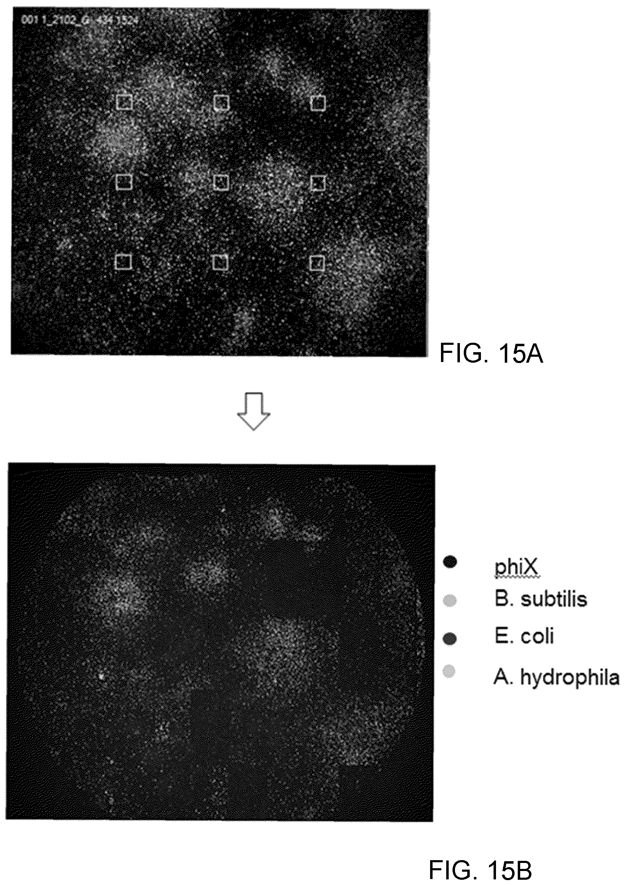

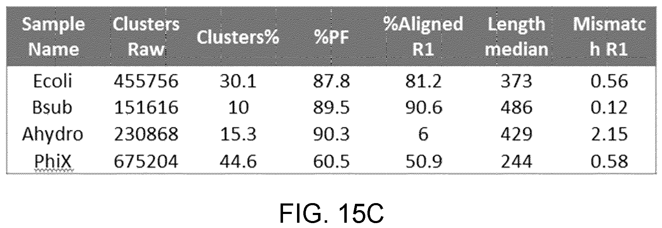

FIGs. 15A-15C depict MiSeq sequencing results of flow cell seeding with hydrogel beads containing *E. coli, B. subtilis,* or *A. hydrophila* bacteria. FIG. 15A is an image that depicts raw data from MiSeq microscopy. FIG. 15B is an image that shows different microbial clusters that aligned to specific genome. For example, the dots are the clusters that aligned to *E*. *coli* genome showing the group distribution pattern. FIG. 15C is a summary table of the pass filter of three different bacterial species.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein.

Embodiments of the compositions, systems, and methods provided herein relate to beads encapsulating genetic materials. The beads may include hydrogel polymers and crosslinkers that are mixed in the presence of a genetic material, and which form hydrogel beads that encapsulate the genetic material. In some embodiments, the genetic material includes nucleic acids, cells, microbiomes, or genomes. The hydrogel beads may include pores that allow diffusion of reagents through the hydrogel bead while retaining the genetic material within the bead, thereby allowing reactions to take place within the beads. Also provided are methods of using the beads encapsulating genetic materials to perform nucleic acid reactions.

Some embodiments relate to methods of preparing a hydrogel bead that encapsulates an entire single cell. In some embodiments, the hydrogel bead encapsulating a single cell can be used to process the cellular genome and perform whole DNA library preparation inside the bead. In some embodiments, the hydrogel bead encapsulating a single cell can be used to process the cellular genomic DNA and mRNA simultaneously, and perform whole DNA library preparation inside the bead. The pore size of the hydrogel can be engineered to allow the diffusion of enzymes, chemicals, and smaller sized primers (< 50bps), while retaining larger nucleic acids (>300bps) such that the intact whole genomic DNA, mRNA, and the produced DNA library may be retained inside the hydrogel beads during the whole process. In some embodiments, specific primers can be chemically linked within the hydrogel bead matrix to hybridize and process specific genomic DNA or mRNA. The DNA library from a single cell can then be released to a specific area, for example, on flow cell surface for library seeding. Subsequently, this results in a spatial distribution of "DNA clusters" on the flow cell originating from individual cells, thus simplifying the read alignment during post processing.

The hydrogel encapsulation approach can be used for other complex library preparation operations, such as for example, single cell genomics/transcriptomics applications, because the approach allows retention of the nucleic acid molecules inside the hydrogel while enabling exchange of reagents to perform various enzymatic/chemical operations on the DNA molecules. This approach was found to enhance the usefulness of single cell genomics for microbial species in comparison to current technology of FACS sorting of microbes into microwells to perform various lysis and library prep workflows and is low throughput (96 or 384 cells per plate). The approach described herein enables hundreds of thousands of microbes to be converted into a barcoded library in an efficient process.

As used herein, the term "reagent" describes an agent or a mixture of two or more agents useful for reacting with, interacting with, diluting, or adding to a sample, and may include agents used in nucleic acid amplification reactions, including, for example buffers, chemicals, enzymes, polymerase, primers having a size of less than 50 base pairs, template nucleic acids, nucleotides, labels, dyes, or nucleases. In some embodiments, the reagent includes lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations.

### Hydrogel Beads Encapsulating Genetic Material

One embodiment includes a bead including a hydrogel polymer and genetic material. As used herein, the term "hydrogel" refers to a substance formed when an organic polymer (natural or synthetic) is cross-linked via covalent, ionic, or hydrogen bonds to create a three-dimensional open-lattice structure that entraps water molecules to form a gel. In some embodiments, the hydrogel may be a biocompatible hydrogel. As used herein, the term "biocompatible hydrogel" refers to a polymer that forms a gel that is not toxic to living cells and allows sufficient diffusion of oxygen and nutrients to entrapped cells to maintain viability. In some embodiments, the hydrogel polymer includes 60-90% fluid, such as water, and 10-30% polymer. In certain embodiments, the water content of hydrogel is about 70-80%.

Hydrogels may be prepared by cross-linking hydrophilic biopolymers or synthetic polymers. Thus, in some embodiments, the hydrogel may include a crosslinker. As used herein, the term "crosslinker" refers to a molecule that can form a three-dimensional network when reacted with the appropriate base monomers. Examples of the hydrogel polymers, which may include one or more crosslinkers, include but are not limited to, hyaluronans, chitosans, agar, heparin, sulfate, cellulose, alginates (including alginate sulfate), collagen, dextrans (including dextran sulfate), pectin, carrageenan, polylysine, gelatins (including gelatin type A), agarose, (meth)acrylate-oligolactide-PEO-oligolactide-(meth)acrylate, PEO-PPO-PEO copolymers (Pluronics), poly(phosphazene), poly(methacrylates), poly(N-vinylpyrrolidone), PL(G)A-PEO-PL(G)A copolymers, poly(ethylene imine), polyethylene glycol (PEG)-thiol, PEG-acrylate, acrylamide, N,N'-bis(acryloyl)cystamine, PEG, polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations thereof. Thus, for example, a combination may include a polymer and a crosslinker, for example polyethylene glycol (PEG)-thiol/PEG-acrylate, acrylamide/N,N'-bis(acryloyl)cystamine (BACy), or PEG/polypropylene oxide (PPO).

In some embodiments, a crosslinker forms a disulfide bond in the hydrogel polymer. As shown in FIG. 7A, a crosslinker may form a disulfide bond, thereby linking hydrogel polymers. In some embodiments, the hydrogel polymers form a hydrogel matrix having pores (for example, a porous hydrogel matrix). These pores are capable of retaining sufficiently large genetic material within the hydrogel bead, but allow small materials, such as reagents, to pass through the pores, thereby passing in and out of the hydrogel beads. In some embodiments, the pore size is finely tuned by varying the ratio of the concentration of polymer to the concentration of crosslinker. In some embodiments, the ratio of polymer to crosslinker is 30:1, 25:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, or 1:30, or a ratio within a range defined by any two of the aforementioned ratios. In some embodiments, additional functions such as DNA primer, or charged chemical groups can be grafted to polymer matrix to meet the requirements of different applications.

As used herein, the term "porosity" means the fractional volume (dimension-less) of a hydrogel that is composed of open space, for example, pores or other openings. Therefore, porosity measures void spaces in a material and is a fraction of volume of voids over the total volume, as a percentage between 0 and 100% (or between 0 and 1). Porosity of the hydrogel may range from 0.5 to 0.99, from about 0.75 to about 0.99, or from about 0.8 to about 0.95.

The hydrogels can have any pore size. As used herein, the term "pore size" refers to a diameter or an effective diameter of a cross-section of the pores. The term "pore size" can also refer to an average diameter or an average effective diameter of a cross-section of the pores, based on the measurements of a plurality of pores. The effective diameter of a cross-section that is not circular equals the diameter of a circular cross-section that has the same cross-sectional area as that of the non-circular cross-section. In some embodiments, the hydrogel can be swollen when the hydrogel is hydrated. The sizes of the pores size can then change depending on the water content in the hydrogel. In some embodiments, the pores of the hydrogel can have a pore of sufficient size to retain genetic material within the hydrogel but allow reagents to pass through.

In some embodiments, the crosslinker is a reversible crosslinker. In some embodiments, a reversible crosslinker is capable of reversibly crosslinking the hydrogel polymer and is capable of being un-crosslinked in the presence of a cleaver. In some embodiments, a crosslinker can be cleaved by the presence of a reducing agent, by elevated temperature, or by an electric field. In some embodiments, the reversible crosslinker may be N,N'-bis(acryloyl)cystamine, a reversible crosslinker for polyacrylamide gels, wherein a disulfide linkage may be broken in the presence of a suitable reducing agent. As shown in FIGs. 6A and 6B, contacting the crosslinker with a reducing agent cleaves the disulfide bonds of the crosslinker, breaking down the hydrogel beads. The hydrogel beads degrade, and release the contents, such as nucleic acids that were retained therein, as shown in FIG. 7C. In some embodiments, the crosslinker is cleaved by increasing the temperature to greater than 50, 55, 60, 65, 70, 75, 80, 85, 90, 95, or 100°C. In some embodiments, the crosslinker is cleaved by contacting the hydrogel beads with a reducing agent. In some embodiments, the reducing agents include phosphine compounds, water soluble phosphines, nitrogen containing phosphines and salts and derivatives thereof, dithioerythritol (DTE), dithiothreitol (DTT) (cis and trans isomers, respectively, of 2,3-dihydroxy-1,4-dithiolbutane), 2-mercaptoethanol or β-mercaptoethanol (BME), 2-mercaptoethanol or aminoethanethiol, glutathione, thioglycolate or thioglycolic acid, 2,3-dimercaptopropanol, tris(2-carboxyethyl)phosphine (TCEP), tris(hydroxymethyl)phosphine (THP), or P-[tris(hydroxymethyl)phosphine] propionic acid (THPP).

In some embodiments, elevating the temperature to increase diffusion or contacting with a reducing agent degrades the crosslinker, thereby releasing encapsulated genetic material from the hydrogel bead.

In some embodiments, the crosslinking of the crosslinker establishes pores within the hydrogel bead. In some embodiments, the size of the pores in the hydrogel beads are regulatable and are formulated to encapsulate genetic material, such as cells or nucleic acids of greater than about 300 base pairs, but to allow smaller particles, such as reagents, or smaller sized nucleic acids of less than about 50 base pairs, such as primers, to pass through the pores. In some embodiments, the reagents including reagents for processing genetic material, such as reagents for isolating nucleic acids from a cell, for amplifying or sequencing nucleic acids, or for preparation of nucleic acid libraries. In some embodiments, reagents include, for example, lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations.

In some embodiments, the hydrogel beads include genetic material. Genetic material, as used herein, refers to cells, microbiomes, or nucleic acids. In some embodiments, the cell is a single cell, including a prokaryotic or a eukaryotic cell. In some embodiments, the cell is a mammalian cell. In some embodiments, the cell is a human cell. In some embodiments, the cell is a bacterial cell. In some embodiments, the genetic material is a viral particle. In some embodiments, the nucleic acid is a long DNA molecule, genomic DNA, viral nucleic acids, bacterial nucleic acids, or mammalian nucleic acids. Any genetic materials may be encapsulated within the hydrogel beads.

### Methods of Making Beads

Some embodiments provided herein relate to methods of making beads encapsulating genetic material. In some embodiments, a hydrogel bead is prepared by vortex assisted emulsion. As used herein, vortex assisted emulsion refers to vortexing of a hydrogel polymer with genetic material in a container, such as in a tube, vial, or reaction vessel, as shown in FIGs. 1A and 1B. The components can be mixed, for example by manual or mechanical vortexing or shaking. In some embodiments, manual mixing results in hydrogel beads that encapsulate genetic material having a size of 2, 3, 4, 5, 10, 15, 20, 25, 30, 35, 40, 45, 50, 55, 60, 65, 70, 75, 80, 85, 90, 95, 100, 110, 120, 130, 140, or 150 µm in diameter, or a size within a range defined by any two of the aforementioned values. In some embodiments, the size of the beads is non-uniform, and thus, the size of the beads includes beads of various diameters.

In some embodiments, the beads are prepared by microfluidic flow techniques. Microfluidic flow includes use of a microfluidic device 200 for assisted gel emulsion generation, as shown in FIG. 2A. In some embodiments, the microfluidic device 200 includes microchannels configured to produce a hydrogel bead 210 of a desired size and configured to encapsulate a selected amount of genetic material per bead. In some embodiments, the microfluidic device 200 has a height of 50, 60, 70, 80, 90, 100, 110, 120, 130, 140, 150, 160, 170, 180, 190, or 200 µm, or a height within a range defined by any two of the aforementioned values. In some embodiments, the microfluidic device 200 includes one or more channels. In some embodiments, the microfluidic device 200 includes a channel 215 for an aqueous stream and a channel 220 for an immiscible fluid. In some embodiments, the width of the one or more channels is identical. In some embodiments, the width of the one or more channels is different. In some embodiments, the width of the one or more channels is 20, 30, 40, 45, 50, 55, 60, 65, 70, 75, 80, 85, 90, 95, 100, 105, 110, 115, 120, 125, 130, 135, 140, 145, or 150 µm, or a width within a range defined by any two of the aforementioned values. In some embodiments, the width of the aqueous channel is 75 µm. In some embodiments, the width of the immiscible fluid channel is 78 µm. One of skill in the art will recognize that the width can vary to finely tune the size of the bead 210. In addition to the size of the microfluidic device 200 and the width of the channels, the flow rate of the aqueous channel and the immiscible fluid channel may also affect the size of the hydrogel beads.

In some embodiments, the flow rate of the solution in the aqueous phase channel is 1, 2, 5, 10, 20, 30, 40, 50, 60, 70, 80, 90, 100, 110, 120, 130, 140, or 150 µL/min, or a rate within a range defined by any two of the aforementioned values. In some embodiments, the flow rate of the immiscible fluid in the immiscible fluid channel is 20, 30, 50, 80, 100, 150, 160, 170, 180, 190, 200, 225, 250, 275, 300, 325, 350, 375, or 400 µL/min, or a rate within a range defined by any two of the aforementioned values. In some embodiments, the solution in the aqueous phase includes a hydrogel polymer, a crosslinker, and genetic material, which flows through the aqueous channel 215 into an immiscible fluid, such as a carrier oil, at a flow rate less than the flow rate of the immiscible fluid, thereby forming droplets. In some embodiments, the immiscible fluid is oil, such as mineral oil, a hydrocarbon oil, a silicon oil, or a polydimethylsiloxane oil, or mixtures thereof. In some embodiments, the hydrogel droplets containing genetic material are formulated in a uniform size distribution. In some embodiments, the size of the hydrogel beads is finely tuned by adjusting the size of the microfluidic device, the size of the one or more channels, or the flow rate of either or both of the aqueous solution or immiscible fluid. In some embodiments, the resulting hydrogel bead has a diameter ranging from 2 to 150 µm, for example, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, or 150 µm, or a diameter within a range defined by any two of the aforementioned values.

In some embodiments, the size and uniformity of the hydrogel bead encapsulating genetic material can be further controlled by contacting hydrogel polymer prior to bead formation with a fluidic modifier, such as with an alcohol, including isopropyl alcohol. As shown in FIG. 3, in the absence of isopropyl alcohol, beads form at a greater diameter than beads formed in the presence of isopropyl alcohol. Isopropyl alcohol influences the fluidic property of the hydrogel polymer, allowing modulation of the hydrogel beads.

In some embodiments, a hydrogel bead, whether prepared by vortex assisted emulsion or microfluidic inertial flow assisted emulsion, encapsulate a single or distinct genetic material. For example, in some embodiments, the bead encapsulates a single cell. In some embodiments, the amount of genetic material within a bead can be controlled by diluting or concentration the genetic material within the inputted sample. The sample including the genetic material is mixed with hydrogel polymer, and the hydrogel polymer containing the genetic material is submitted to vortex assisted emulsion or microfluidic flow assisted emulsion, as described herein.

In some embodiments, the hydrogel beads are functionalized with a nucleotide. In some embodiments, the nucleotide is an oligonucleotide or polyT nucleotide. In some embodiments, the nucleotide is bound to the hydrogel bead, and the functionalized bead can be used for targeted capture of a nucleotide of interest.

### Methods of Processing Genetic Material within Hydrogel Beads

Some embodiments provided herein relate to methods of processing genetic material within a bead. In some embodiments, genetic material encapsulated within a hydrogel bead is contacted with one or more reagents for nucleic acid processing. In some embodiments, the genetic material is retained within the hydrogel beads, and reagents are able to pass through the pores of the hydrogel beads. In some embodiments, reagents can include lysis agents, nucleic acid purification agents, DNA amplification agents, tagmentation agents, PCR agents, or other agents used in processing of genetic materials. Thus, the hydrogel beads provide a microenvironment for controlled reactions of genetic materials within the hydrogel beads by allowing a barrier for reagents to pass in and out of the hydrogel beads, while retaining the genetic material itself within the beads.

As used herein, the term "tagmentation" refers to the modification of DNA by a transposome complex comprising transposase enzyme complexed with adaptors comprising transposon end sequence. Tagmentation results in the simultaneous fragmentation of the DNA and ligation of the adaptors to the 5' ends of both strands of duplex fragments. Following a purification step to remove the transposase enzyme, additional sequences can be added to the ends of the adapted fragments, for example by PCR, ligation, or any other suitable methodology known to those of skill in the art.

In some embodiments, entire DNA library preparation can be accomplished seamlessly inside the hydrogel beads with multiple reagent exchanges by passing through the porous hydrogel while retaining the gDNA and its library products within the hydrogel matrix. The hydrogel may be resistant to high temperatures up to 95°C for several hours to support different biochemical reactions.

In some embodiments, the hydrogel bead encapsulating a cell or viral particle is treated to purify and isolate nucleic acids from the cell or particle. Thus, for example the hydrogel bead is contacted with a lysis buffer. As used herein, "lysis" means perturbation or alteration to a cell wall or viral particle facilitating access to or release of the cellular RNA or DNA. Neither complete disruption nor breakage of the cell wall is an essential requirement for lysis. By the term "lysis buffer" is meant a buffer that contains at least one lysing agent. Typical enzymatic lysing agents include, but are not limited to, lysozyme, glucolase, zymolose, lyticase, proteinase K, proteinase E, and viral endolysins and exolysins. Thus, for example, lysis of cells in the beads may be performed by introducing lysing agents, such as lysozyme and proteinase K into the hydrogel beads. The gDNA from the cells is now contained within the beads. In some embodiments, following lysis treatment, isolated nucleic acid is retained within the hydrogel bead, and may be used for further processing.

As used herein, the terms "isolated," "to isolate," "isolation," "purified," "to purify," "purification," and grammatical equivalents thereof as used herein, unless specified otherwise, refer to the reduction in the amount of at least one contaminant (such as protein and/or nucleic acid sequence) from a sample or from a source (e.g., a cell) from which the material is isolated. Thus purification results in an "enrichment," for example, an increase in the amount of a desirable protein and/or nucleic acid sequence in the sample.

Following lysis and isolation of nucleic acids, amplification may be performed, such as multiple displacement amplification (MDA), which is a widely used technique for amplifying low quantities of DNA, especially from single cells. In some embodiments, the encapsulated nucleic acids are amplified, sequenced, or used for the preparation of nucleic acid libraries. As used herein, the terms "amplify" or "amplified" "amplifying" as used in reference to a nucleic acid or nucleic acid reactions, refer to in vitro methods of making copies of a particular nucleic acid, such as a target nucleic acid, or a nucleic acid encapsulated within a bead, for example, by an embodiment of the present invention. Numerous methods of amplifying nucleic acids are known in the art, and amplification reactions include polymerase chain reactions, ligase chain reactions, strand displacement amplification reactions, rolling circle amplification reactions, multiple annealing and looping based amplification cycles (MALBAC), transcription-mediated amplification methods such as NASBA, loop mediated amplification methods (e.g., "LAMP" amplification using loop-forming sequences. The nucleic acid that is amplified can be DNA comprising, consisting of, or derived from DNA or RNA or a mixture of DNA and RNA, including modified DNA and/or RNA. The products resulting from amplification of a nucleic acid molecule or molecules (for example, "amplification products"), whether the starting nucleic acid is DNA, RNA or both, can be either DNA or RNA, or a mixture of both DNA and RNA nucleosides or nucleotides, or they can comprise modified DNA or RNA nucleosides or nucleotides. A "copy" does not necessarily mean perfect sequence complementarity or identity to the target sequence. For example, copies can include nucleotide analogs such as deoxyinosine or deoxyuridine, intentional sequence alterations (such as sequence alterations introduced through a primer comprising a sequence that is hybridizable, but not complementary, to the target sequence, and/or sequence errors that occur during amplification.

The encapsulated nucleic acids that are isolated within the hydrogel beads can be amplified according to any suitable amplification methodology known in the art. In some embodiments, the encapsulated nucleic acids are amplified within the bead. In some embodiments, the bead is captured on a solid support and degraded, wherein the encapsulated nucleic acids are released onto the solid support, and the nucleic acids are amplified on the solid support.

In some embodiments, the encapsulated nucleic acids are amplified within the hydrogel beads. For example, in some embodiments, amplification primers and enzymes pass through the pores of the hydrogel beads and hybridize to the encapsulated nucleic acids.

It will be appreciated that any of the amplification methodologies described herein or generally known in the art can be utilized with universal or target-specific primers to amplify encapsulated nucleic acids. Suitable methods for amplification include, but are not limited to, the polymerase chain reaction (PCR), strand displacement amplification (SDA), transcription mediated amplification (TMA) and nucleic acid sequence based amplification (NASBA), as described in U.S. Pat. No. 8,003,354, which is incorporated herein by reference in its entirety. The above amplification methods can be employed to amplify one or more nucleic acids of interest. For example, PCR, including multiplex PCR, SDA, TMA, NASBA and the like can be utilized to amplify encapsulated nucleic acids. In some embodiments, primers directed specifically to the nucleic acid of interest are included in the amplification reaction.

Other suitable methods for amplification of nucleic acids can include oligonucleotide extension and ligation, rolling circle amplification (RCA) (Lizardi et al., Nat. Genet. 19:225-232 (1998), which is incorporated herein by reference) and oligonucleotide ligation assay (OLA) technologies (See generally U.S. Pat. Nos. 7,582,420, 5,185,243, 5,679,524 and 5,573,907; EP 0 320 308 B1; EP 0 336 731 B1; EP 0 439 182B1; WO 90/01069; WO 89/12696; and WO 89/09835, all of which are incorporated by reference). It will be appreciated that these amplification methodologies can be designed to amplify encapsulated nucleic acids. For example, in some embodiments, the amplification method can include ligation probe amplification or oligonucleotide ligation assay (OLA) reactions that contain primers directed specifically to the nucleic acid of interest. In some embodiments, the amplification method can include a primer extension-ligation reaction that contains primers directed specifically to the nucleic acid of interest, and which are capable of passing through the hydrogel pores. As a non-limiting example of primer extension and ligation primers that can be specifically designed to amplify a nucleic acid of interest, the amplification can include primers used for the GoldenGate assay (Illumina, Inc., San Diego, Calif.) as exemplified by U.S. Pat. Nos. 7,582,420 and 7,611,869, each of which is incorporated herein by reference in its entirety. In each of the methods described, the reagents and components involved in the nucleic acid reaction are capable of passing through the pores of the hydrogel bead while retaining the nucleic acid itself within the bead.

In some embodiments, the encapsulated nucleic acids are amplified using cluster amplification methodologies as exemplified by the disclosures of U.S. Pat. Nos. 7,985,565 and 7,115,400, the contents of each of which are incorporated herein by reference in their entirety. The incorporated materials of U.S. Pat. Nos. 7,985,565 and 7,115,400 describe methods of nucleic acid amplification which allow amplification products to be immobilized on a solid support in order to form arrays comprised of clusters or "colonies" of immobilized nucleic acid molecules. Each cluster or colony on such an array is formed from a plurality of identical immobilized polynucleotide strands and a plurality of identical immobilized complementary polynucleotide strands. The arrays so-formed are generally referred to herein as "clustered arrays". The products of solid-phase amplification reactions such as those described in U.S. Pat. Nos. 7,985,565 and 7,115,400 are so-called "bridged" structures formed by annealing of pairs of immobilized polynucleotide strands and immobilized complementary strands, both strands being immobilized on the solid support at the 5' end, preferably via a covalent attachment. Cluster amplification methodologies are examples of methods wherein an immobilized nucleic acid template is used to produce immobilized amplicons. Other suitable methodologies can also be used to produce immobilized amplicons from immobilized DNA fragments produced according to the methods provided herein. For example one or more clusters or colonies can be formed via solid-phase PCR whether one or both primers of each pair of amplification primers are immobilized. In some embodiments, the encapsulated nucleic acids are amplified within the beads, and then deposited in an array or on a solid support in a cluster. For example, as shown in FIG. 12, hydrogel beads encapsulating nucleic acids were processed and the nucleic acids contained therein were used to establish two different indexed DNA libraries. Two types of hydrogel beads were prepared containing PhiX library, one with Index 2 (CGATGT) and other with Index 4 (TGACCA). Hydrogel beads with Index 2 and Index 4 are mixed and loaded into the flow cell. The beads were contacted with a surface, and degraded, and the library was deposited on the surface. The micrographs depict nucleic acid imaging, showing clusters that were formed based on the separate environments within separate beads. The image shows the spatial separation between the Index 2 and Index 4 libraries, indicating that the libraries originated from different hydrogel beads. Scale bar = 50 µm.

Additional amplification methods include isothermal amplification. Exemplary isothermal amplification methods that can be used include, but are not limited to, multiple displacement amplification (MDA) as exemplified by, for example Dean et al., Proc. Natl. Acad. Sci. USA 99:5261-66 (2002) or isothermal strand displacement nucleic acid amplification exemplified by, for example U.S. Pat. No. 6,214,587, each of which is incorporated herein by reference in its entirety. Other non-PCR-based methods that can be used in the present disclosure include, for example, strand displacement amplification (SDA) which is described in, for example Walker et al., Molecular Methods for Virus Detection, Academic Press, Inc., 1995; U.S. Pat. Nos. 5,455,166, and 5,130,238, and Walker et al., Nucl. Acids Res. 20:1691-96 (1992) or hyperbranched strand displacement amplification which is described in, for example Lage et al., Genome Research 13:294-307 (2003), each of which is incorporated herein by reference in its entirety. Isothermal amplification methods can be used with the strand-displacing Phi 29 polymerase or Bst DNA polymerase large fragment, 5'->3' exo- for random primer amplification of genomic DNA. The use of these polymerases takes advantage of their high processivity and strand displacing activity. High processivity allows the polymerases to produce fragments that are 10-20 kb in length. As set forth above, smaller fragments can be produced under isothermal conditions using polymerases having low processivity and strand-displacing activity such as Klenow polymerase. Additional description of amplification reactions, conditions and components are set forth in detail in the disclosure of U.S. Pat. No. 7,670,810, which is incorporated herein by reference in its entirety. In some embodiments, the polymerases, reagents, and components required to perform these amplification reactions are capable of passing through the pores of the hydrogel beads to interact with the encapsulated nucleic acids, thereby amplifying the nucleic acids within the hydrogel beads. In some embodiments, random hexamers are annealed to the denatured DNA followed by strand displacement synthesis at a constant temperature in the presence of a catalyzing enzyme, Phi 29. This results in DNA amplification within the beads as confirmed by an increase in the fluorescence intensity (DNA was stained with SYTOX) after MDA (FIG. 9, panel a). Independently, Nextera based tagmentation after lysis and clean up and subsequent gDNA amplification via PCR as indicated by a substantial increase in fluorescence intensity within the beads after Nextera tagmentation and PCR may also be performed (FIG. 9, panel b). After this Nextera library preparation, the gel beads may be heated to 80°C for 3 minutes to release the contents of the beads namely, the sequencing ready library products from single cells as shown in FIG. 9, panel c.

Another nucleic acid amplification method that is useful in the present disclosure is Tagged PCR which uses a population of two-domain primers having a constant 5' region followed by a random 3' region as described, for example, in Grothues et al. Nucleic Acids Res. 21(5):1321-2 (1993), incorporated herein by reference in its entirety. The first rounds of amplification are carried out to allow a multitude of initiations on heat denatured DNA based on individual hybridization from the randomly-synthesized 3' region. Due to the nature of the 3' region, the sites of initiation are contemplated to be random throughout the genome. Thereafter, the unbound primers can be removed and further replication can take place using primers complementary to the constant 5' region.

In some embodiments, the encapsulated nucleic acids are sequenced in full or in part within the hydrogel beads. The encapsulated nucleic acids can be sequenced according to any suitable sequencing methodology, such as direct sequencing, including sequencing by synthesis, sequencing by ligation, sequencing by hybridization, nanopore sequencing and the like. As shown schematically in FIG. 8B, single cells encapsulated within a hydrogel bead may be processed for single cell sequencing.

One sequencing methodology is sequencing-by-synthesis (SBS). In SBS, extension of a nucleic acid primer along a nucleic acid template (e.g. a target nucleic acid or amplicon thereof) is monitored to determine the sequence of nucleotides in the template. The underlying chemical process can be polymerization (e.g. as catalyzed by a polymerase enzyme). In a particular polymerase-based SBS embodiment, fluorescently labeled nucleotides are added to a primer (thereby extending the primer) in a template dependent fashion such that detection of the order and type of nucleotides added to the primer can be used to determine the sequence of the template.

One or more amplified encapsulated nucleic acids can be subjected to an SBS or other detection technique that involves repeated delivery of reagents in cycles. For example, to initiate a first SBS cycle, one or more labeled nucleotides, DNA polymerase, etc., can be flowed into/through a hydrogel bead that houses one or more amplified nucleic acid molecules. Those sites where primer extension causes a labeled nucleotide to be incorporated can be detected. Optionally, the nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for embodiments that use reversible termination, a deblocking reagent can be delivered to the flow cell (before or after detection occurs). Washes can be carried out between the various delivery steps. The cycle can then be repeated n times to extend the primer by n nucleotides, thereby detecting a sequence of length n. Exemplary SBS procedures, fluidic systems and detection platforms that can be readily adapted for use with amplicons produced by the methods of the present disclosure are described, for example, in Bentley et al., Nature 456:53-59 (2008), WO 04/018497; U.S. Pat. No. 7,057,026; WO 91/06678; WO 07/123744; U.S. Pat. No. 7,329,492; U.S. Pat. No. 7,211,414; U.S. Pat. No. 7,315,019; U.S. Pat. No. 7,405,281, and US 2008/0108082, each of which is incorporated herein by reference.

Other sequencing procedures that use cyclic reactions can be used, such as pyrosequencing. Pyrosequencing detects the release of inorganic pyrophosphate (PPi) as particular nucleotides are incorporated into a nascent nucleic acid strand (Ronaghi, et al., Analytical Biochemistry 242(1), 84-9 (1996); Ronaghi, Genome Res. 11(1), 3-11 (2001); Ronaghi et al. Science 281(5375), 363 (1998); U.S. Pat. No. 6,210,891; U.S. Pat. No. 6,258,568 and U.S. Pat. No. 6,274,320, each of which is incorporated herein by reference). In pyrosequencing, released PPi can be detected by being immediately converted to adenosine triphosphate (ATP) by ATP sulfurylase, and the level of ATP generated can be detected via luciferase-produced photons. Thus, the sequencing reaction can be monitored via a luminescence detection system. Excitation radiation sources used for fluorescence based detection systems are not necessary for pyrosequencing procedures. Useful fluidic systems, detectors and procedures that can be adapted for application of pyrosequencing to amplicons produced according to the present disclosure are described, for example, in WIPO Pat. App. Ser. No. PCT/US11/57111, US 2005/0191698 A1, U.S. Pat. No. 7,595,883, and U.S. Pat. No. 7,244,559, each of which is incorporated herein by reference.

Some embodiments can utilize methods involving the real-time monitoring of DNA polymerase activity. For example, nucleotide incorporations can be detected through fluorescence resonance energy transfer (FRET) interactions between a fluorophore-bearing polymerase and γ-phosphate-labeled nucleotides, or with zero mode waveguides (ZMWs). Techniques and reagents for FRET-based sequencing are described, for example, in Levene et al. Science 299, 682-686 (2003); Lundquist et al. Opt. Lett. 33, 1026-1028 (2008); Korlach et al. Proc. Natl. Acad. Sci. USA 105, 1176-1181 (2008), the disclosures of which are incorporated herein by reference.

Some SBS embodiments include detection of a proton released upon incorporation of a nucleotide into an extension product. For example, sequencing based on detection of released protons can use an electrical detector and associated techniques that are commercially available. Examples of such sequencing systems are pyrosequencing (e.g. commercially available platform from 454 Life Sciences a subsidiary of Roche), sequencing using γ-phosphate-labeled nucleotides (e.g. commercially available platform from Pacific Biosciences) and sequencing using proton detection (e.g. commercially available platform from Ion Torrent subsidiary of Life Technologies) or sequencing methods and systems described in US 2009/0026082 A1; US 2009/0127589 A1; US 2010/0137143 A1; or US 2010/0282617 A1, each of which is incorporated herein by reference. Methods set forth herein for amplifying target nucleic acids using kinetic exclusion can be readily applied to substrates used for detecting protons. More specifically, methods set forth herein can be used to produce clonal populations of amplicons that are used to detect protons.

Another sequencing technique is nanopore sequencing (see, for example, Deamer et al. Trends Biotechnol. 18, 147-151 (2000); Deamer et al. Acc. Chem. Res. 35:817-825 (2002); Li et al. Nat. Mater. 2:611-615 (2003), the disclosures of which are incorporated herein by reference). In some nanopore embodiments, the target nucleic acid or individual nucleotides removed from a target nucleic acid pass through a nanopore. As the nucleic acid or nucleotide passes through the nanopore, each nucleotide type can be identified by measuring fluctuations in the electrical conductance of the pore. (U.S. Pat. No. 7,001,792; Soni et al. Clin. Chem. 53, 1996-2001 (2007); Healy, Nanomed. 2, 459-481 (2007); Cockroft et al. J. Am. Chem. Soc. 130, 818-820 (2008), the disclosures of which are incorporated herein by reference).

Exemplary methods for array-based expression and genotyping analysis that can be applied to detection according to the present disclosure are described in U.S. Pat. No. 7,582,420; 6,890,741; 6,913,884 or 6,355,431 or US Pat. Pub. Nos. 2005/0053980 A1; 2009/0186349 A1 or US 2005/0181440 A1, each of which is incorporated herein by reference.

In the methods of isolating nucleic acids, amplification, and sequencing as described herein, various reagents are used for nucleic acid isolation and preparation. Such reagents may include, for example, lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations. These reagents pass through the pores of the hydrogel beads, whereas the genetic material is retained within the hydrogel beads. An advantage of the methods set forth herein is that they provide for an encapsulated microenvironment for the processing of nucleic acids within a hydrogel bead. This enables single cell processing for rapid and efficient processing of a target nucleic acid.

Adaptors can include sequencing primer sites, amplification primer sites, and indexes. As used herein an "index" can include a sequence of nucleotides that can be used as a molecular identifier and/or barcode to tag a nucleic acid, and/or to identify the source of a nucleic acid. In some embodiments, an index can be used to identify a single nucleic acid, or a subpopulation of nucleic acids. In some embodiments, nucleic acid libraries can be prepared within a hydrogel bead. As shown schematically in FIG. 8A, a single cell encapsulated within a hydrogel bead may be used for combinatorial indexing of the single cells, for example, using a contiguity preserving transposition (CPTSeq) approach. In some embodiments, as shown schematically in FIG. 8C, DNA from a single cell may be barcoded by encapsulation of single cells after WGA amplification with another bead carrying barcoded transposons and dissolving the gel matrix by contacting it with a reducing agent, for example, to release genomic DNA for barcoding.

Embodiments of the "spatial indexing" methods and techniques described herein shortens data analysis and simplifies the process of library preparation from single cells and long DNA molecules. Existing protocols for single cell sequencing requires efficient physical separation of the cells and uniquely barcoding each isolated cell and pooling everything back together to do sequencing. Current protocols for synthetic long reads also requires cumbersome barcoding steps, and pooling each barcoded fragments together for sequencing and letting data analysis to distinguish genetic information coming from each barcoded cell. During these long processes there is also loss of genetic material which causes dropouts in the sequences. Embodiments described herein not only shorten the process but also increase data resolution for single cells. Furthermore, embodiments provided herein simplify the assembly of genomes of new organisms. Embodiments described herein may be used to reveal rare genetic variations and co-occurrence of mutations. In some embodiments, DNA library confined in the hydrogel beads until release provide the opportunity to control the size of the fragments that is released on the surface by controlling the release process and hydrogel formulation.

In some embodiments, the library may be amplified using primer sites in the adaptor sequences, and sequenced using sequencing primer sites in the adaptor sequences. In some embodiments the adaptor sequences can include indexes to identify the source of the nucleic acids. The efficiency of subsequent amplification steps can be reduced by the formation of primer-dimers. To increase the efficiency of subsequent amplification steps, non-ligated single-stranded adaptors can be removed from ligation products.

### Preparing Nucleic Acid Libraries with Hydrogel Beads

Some embodiments of the systems, methods and compositions provided herein include methods in which adaptors are ligated to target nucleic acids. Adaptors can include sequencing primer binding sites, amplification primer binding sites, and indexes. For example, an adaptor can include a P5 sequence, a P7 sequence, or a complement thereof. As used herein a P5 sequence comprises a sequence defined by SEQ ID NO: 1 (AATGATACGGCGACCACCGA) and a P7 sequence comprises a sequence defined by SEQ ID NO: 2 (CAAGCAGAAGACGGCATACGA). In some embodiments, the P5 or P7 sequence can further include a spacer polynucleotide, which may be from 1 to 20, such as 1 to 15, or 1 to 10, nucleotides, such as 2, 3, 4, 5, 6, 7, 8, 9, or 10 nucleotides in length. In some embodiments, the spacer includes 10 nucleotides. In some embodiments, the spacer is a polyT spacer, such as a 10T spacer. Spacer nucleotides may be included at the 5' ends of polynucleotides, which may be attached to a suitable support via a linkage with the 5' end of the polynucleotide. Attachment can be achieved through a sulphur-containing nucleophile, such as phosphorothioate, present at the 5' end of the polynucleotide. In some embodiments, the polynucleotide will include a polyT spacer and a 5' phosphorothioate group. Thus, in some embodiments, the P5 sequence is 5'phosphorothioate-TTTTTTTTTTAATGATACGGCGACCACCGA-3', SEQ ID NO: 3 and in some embodiments, the P7 sequence is 5'phosphorothioate-TTTTTTTTTTAATGATACGGCGACCACCGA-3', SEQ ID NO. 4.

Indexes can be useful to identify the source of a nucleic acid molecule. In some embodiments, an adaptor can be modified to prevent the formation of concatemers, for example by the addition of blocking groups that prevent extension of the adaptor at one or both ends. Examples of 3' blocking groups include a 3'-spacer C3, a dideoxynucleotide, and attachment to a substrate. Examples of 5' blocking groups include a dephosphorylated 5' nucleotide, and attachment to a substrate.

Adaptors include nucleic acids, such as single-stranded nucleic acids. Adaptors can include short nucleic acids having a length less than, greater than, or equal to about 5 nucleotides, 10 nucleotides, 20 nucleotides, 30 nucleotides, 40 nucleotides, 50 nucleotides, 60 nucleotides, 70 nucleotides, 80 nucleotides, 90 nucleotides, 100 nucleotides, or a range between any two of the foregoing sizes. In some embodiments, the adaptors are of sufficient size to pass through the pores of the hydrogel beads. Target nucleic acids include DNA, such as genomic or cDNA; RNA, such as mRNA, sRNA or rRNA; or a hybrid of DNA and RNA. The nucleic acid can be isolated from a single cell encapsulated within a hydrogel bead. A nucleic acid can contain phosphodiester bonds, and can include other types of backbones, comprising, for example, phosphoramide, phosphorothioate, phosphorodithioate, O-methylphosphoroamidite and peptide nucleic acid backbones and linkages. A nucleic acid can contain any combination of deoxyribo- and ribonucleotides, and any combination of bases, including uracil, adenine, thymine, cytosine, guanine, inosine, xanthanine, hypoxanthanine, isocytosine, isoguanine, and base analogs such as nitropyrrole (including 3-nitropyrrole) and nitroindole (including 5-nitroindole). In some embodiments, a nucleic acid can include at least one promiscuous base. A promiscuous base can base-pair with more than one different type of base and can be useful, for example, when included in oligonucleotide primers or inserts that are used for random hybridization in complex nucleic acid samples such as genomic DNA samples. An example of a promiscuous base includes inosine that may pair with adenine, thymine, or cytosine. Other examples include hypoxanthine, 5-nitroindole, acylic 5-nitroindole, 4-nitropyrazole, 4-nitroimidazole and 3-nitropyrrole. Promiscuous bases that can base-pair with at least two, three, four or more types of bases can be used.

Target nucleic acids can include a sample in which the average size of a nucleic acid in the sample is less than, greater than, or equal to about 2 kb, 1 kb, 500 bp, 400 bp, 200 bp, 100 bp, 50 bp, or a range between any two of the foregoing sizes. In some embodiments, the average size of a nucleic acid in the sample is less than, greater than, or equal to about 2000 nucleotides, 1000 nucleotides, 500 nucleotides, 400 nucleotides, 200 nucleotides, 100 nucleotides, 50 nucleotides, or a range between any two of the foregoing sizes. In some embodiments, the nucleic acid is of sufficient size that the nucleic acid is entrapped within the hydrogel bead such that it cannot pass through the pores of the hydrogel bead.

An example method includes dephosphorylating the 5' ends of target nucleic acids to prevent the formation of concatemers in subsequent ligation steps; ligating first adaptors to the 3' ends of the dephosphorylated targets using a ligase, in which the 3' ends of the first adaptors are blocked; re-phosphorylating of the 5' ends of the ligated targets; ligating a second adaptor to the 5' ends of the dephosphorylated targets using the single-stranded ligase, in which the 5' ends of the second adaptors are non-phosphorylated.

Another example includes partial digestion of the nucleic acid with a 5' exonuclease to form a double-stranded nucleic acid with single-stranded 3' overhangs. An adaptor containing a 3' blocking group can be ligated to the 3' ends of double-stranded nucleic acid with 3' overhangs. The double-stranded nucleic acid with 3' overhangs with ligated adaptors can be dehybridized to form single-stranded nucleic acids. An adaptor containing a non-phosphorylated 5' end can be ligated to the 5' end of the single-stranded nucleic acid.

Methods to dephosphorylate nucleic acids, such as the 5' nucleotide of a nucleic acid include contacting a nucleic acid with a phosphatase. Examples of phosphatases include calf intestinal phosphatase, shrimp alkaline phosphatase, antarctic phosphatase, and APEX alkaline phosphatase (Epicentre).

Methods to ligate nucleic acids include contacting nucleic acids with a ligase. Examples of ligases include T4 RNA ligase 1, T4 RNA ligase 2, RtcB ligase, Methanobacterium RNA ligase, and TS2126 RNA ligase (CIRCLIGASE).

Methods to phosphorylate nucleic acids, such as the 5' nucleotide of a nucleic acid include contacting a nucleic acid with a kinase. Examples of kinases include T4 polynucleotide kinase.

Embodiments provided herein relate to preparing nucleic acids libraries in a hydrogel bead, such that the nucleic acid library is prepared in a single reaction volume.

Embodiments of the systems and methods provided herein include kits, containing any one or more of the hydrogel polymers, crosslinkers, or microfluidic devices for preparing hydrogel beads that encapsulate genetic material, and further including components useful for processing of the genetic material, including reagents for cell lysis, and nucleic acid amplification and sequencing, or for nucleic acid library preparation, including lysozyme, proteinase K, random hexamers, polymerase (for example, Φ29 DNA polymerase, Taq polymerase, Bsu polymerase), transposase (for example, Tn5), primers (for example, P5 and P7 adaptor sequences), ligase, catalyzing enzyme, deoxynucleotide triphosphates, buffers, or divalent cations as described herein, and as used for the respective processing of genetic material.

### EXAMPLES

### Example 1-Preparation of Hydrogel Beads

The following example demonstrates an embodiment of preparing hydrogel beads encapsulating microbial cells using manual vortexing and microfluidic droplet generators.

Samples containing one or more of *E. coli, B. subtilis,* and *A. hydrophila* stored at -80°C were thawed at room temperature. 100 µL of each bacterial solution was transferred to a sterile 1.7 mL tube, and the sample was washed once with 1 mL 0.85% NaCl. The sample was pelleted and wash solution is removed. The bacteria pellet was saved for mixture with the hydrogel solution described as follows.

A hydrogel solution of 12% was prepared from 40% acrylamide/bis 19:1 (BioRad 9161-0144) diluted with deionized water. A 40% (w/v) acrylamide/N,N'-bis(acryloyl)cystamine (BACy) (19:1) monomer stock solution (3.8 g of acrylamide, 0.2 g of BACy, and 6 mL of double distilled (dd) H₂O) was prepared. The mixture was brought to a final volume of 10 mL. To prepare the solution, the acrylamide was dissolved in 6 mL of ddH₂O, and the resulting solution was used to dissolve the BACy. The dissolution of the monomers is endothermic, so heating the mixture slightly will help completely dissolve the monomers. The hydrogel solution was maintained at 4°C until use. 35 µL of saturated potassium persulfate solution (KPS; Sigma) was added to 200 µL of the 12% hydrogel solution and mixed well. The 235 µL hydrogel-KPS solution was added to each tube containing a bacteria cell pellet to resuspend the cells. The hydrogel-bacterial solution was then loaded into 600 µL mineral oil with surfactant (4.5% Span 80, 0.4% Tween 20, and 0.05% Triton X-100). The solution was vortexed for 30 seconds to generate droplet emulsion, and 25 µL of tetramethylethylenediamine (TEMED; Sigma) was immediately added, followed by another 30 seconds of vortexing.

To make a 10% gel with acrylamideBACy ratio of 19:1 (2 mL), 0.96 mL of ddH₂O, 0.5 mL of 40% acrylamideBACy (19:1), 0.5 mL of 10x tris/borate/EDTA buffer (TBE), 20 µL of TEMED and 20 µL of KPS (saturated) were mixed.

To make a 5% gel with acrylamideBACy ratio of 19:1 (2 mL), 1.21 mL of ddH₂O, 0.25 mL of 40% acrylamideBACy (19:1), 0.5 mL of 10x TBE, 20 µL of TEMED and 20 µL of KPS (saturated) were mixed. The reaction was allowed to polymerize until gelation (~3-6 min).

Hydrogel beads encapsulating bacterial cells were then generated. After incubation for 15 minutes to allow the hydrogel beads to fully cross-link, about 900 µL of petroleum ether was added to each tube. Tubes were vortexed to wash away the oil, and supernatant was removed. The hydrogel beads were washed by adding 1 mL of PR2 to each tube, followed by vortexing, and spinning down the beads. The supernatant was removed, and the beads were washed three times with 1 mL of PR2. The beads were resuspended in 1 mL PR2. Hydrogel beads in PR2 can be stored in 4°C for at least 3 weeks.

The method described above using manual emulsion can be used to generate hydrogel beads having a size distribution of from about 2 µm to about 100 µm in diameter.

To generate hydrogel beads of a uniform size distribution, microfluidic droplet generators can be used, such as the generators illustrated in FIG. 2. To form hydrogel beads of a uniform size distribution, an aqueous solution containing a hydrogel polymer and a bacterial cell was introduced into mineral oil in a microfluidic droplet generator. The microfluidic device had a height of 120 µm, with an aqueous channel width of 75 µm and a carrier oil channel width of 78 µm. With this chip, flow rates of 60 µL/min for the aqueous channel and 300 µL/min for the carrier oil channel were used. Using this device, hydrogel beads of approximately 90 µm in diameter were generated. The size of the beads can be finely tuned by adjusting the channel widths, the microfluidic device size, and the flow rates.

In addition, the bead diameter can be finely tuned with the addition of isopropyl alcohol. As shown in FIG. 3, isopropyl alcohol can be added to the hydrogel solution in a specified amount (ranging from 0% to about 30% v/v), to form hydrogel beads of a particular diameter. As shown in FIG. 3, increasing the amount of isopropyl alcohol decreases the hydrogel bead diameter, from 130 µm (with 0% isopropyl alcohol) to about 75 µm in diameter (with 28% isopropyl alcohol).

Hydrogel beads can also be finely tuned to encapsulate a desired quantity of cells. As shown in FIGs. 4A and 4B, numerous stained bacterial cells were found to be present in each hydrogel bead of about 100 µm (Fig 4A), whereas only a single cell, or a few cells, were loaded in each hydrogel bead of about 10 µm (Fig. 4B).

In addition, the rate of gelation of hydrogel beads can also determine the quantity of cells in each hydrogel bead. Hydrogel beads were prepared in the presence of NIH-3T3 mouse fibroblast cells. As shown in FIGs. 6A and 6B fast gelation of hydrogel beads resulted in a single cell per bead (FIG. 6A), whereas slow gelation results in a few cells per bead (FIG. 6B).

### Example 2-Nucleotide Functionalized Hydrogel Beads

The following example demonstrates the method for functionalizing hydrogel beads from Example 1 with nucleotides.

DNA functionalized hydrogel beads were obtained using copper-catalyzed azide-alkyne cycloaddition (CuAAC) chemistry, by reacting azide functionalized hydrogel beads with alkyne functionalized DNA (FIG. 5A). For the synthesis of azide functionalized hydrogel beads, hydrogel precursor mix including, monomers, crosslinkers, and radical generators were mixed and monodisperse hydrogel beads were generated with microfluidics. The hydrogel beads were doped with BrAPA in a concentration ranging from 0% to 8%. The bromides were subsequently azidified to convert the Br groups to azide groups (N3). The azidified hydrogels were then reacted with alkyne-modified oligonucleotides using CuAAC chemistry. The hydrogel beads without azidification did not have any oligonucleotides grafted onto the beads (FIG. 5B, "Original"). On the other hand, hydrogel beads that were azidified had oligonucleotides grafted onto the hydrogel beads (FIG. 5B, "Azidified (N3)"). The density of the oligonucleotides grafted directly correlated to the percentage of BrAPA added in the hydrogel precursor mix.

These oligonucleotide functionalized beads were used to capture mRNA from cells. The hydrogel beads were functionalized with a polyT oligonucleotide (FIG. 5C). The polyT functionalized hydrogel beads were mixed with cells. The cells were subsequently lysed and the beads were collected and washed. Second strand synthesis was performed to make cDNA from the captured mRNA. As shown in FIG. 5D, the electrochromatograms show the trace of the cDNA with its complement (right curve), and the cDNA duplex were then tagmented with Nextera. The tagmented DNA (left curve) shifted to a lower molecular weight as expected due to the tagmentation.

### Example 3-Release of Genetic Material from Hydrogel Beads

The following example demonstrates the ability of the hydrogel beads having genetic material encapsulated therein to release the contents upon reversible cleavage of the crosslinker.

Hydrogel beads encapsulating bacterial cells were prepared according to Example 1. The hydrogel beads include bisacrylamide crosslinkers containing disulfide bonds. The beads were dye-stained for visualization of bacterial cells. As shown in FIG. 6C, hydrogel beads in the absence of a reducing agent were capable of retaining bacterial cells. However, upon contact with a reducing agent, in this case THP, the bacteria were released from the hydrogel beads.

### Example 4-Nucleic Acid Library Preparation in Hydrogel Beads

The following example demonstrates the process for sequencing nucleic acids from genetic material encapsulated within hydrogel beads.

Hydrogel beads as prepared in Example 1 were obtained and nucleic acids were isolated as follows. 50 µL solution of the hydrogel bead encapsulating *E. coli* was transferred to a 200 µL tube (with an approximate 50 beads/µL). The beads were spun down and supernatant removed. 100 µL of bacterial lysis reagent (Thermo Fisher Scientific, 0.5 mg lysozyme in 100 µL resuspension buffer from Charge Switch kit) was added to the tube, and incubated at 37°C for 10 minutes. 500 µL of proteinase K reagent was then added, and incubated at 55°C for 20 minutes to fully digest the proteins. At this step, the *E. coli* gDNA was exposed but pinned inside the hydrogel matrix. The beads retaining bacterial gDNA were washed with PR2 and the hydrogel beads recollected. Tn5 transposome reagent (Nextera, Illumina, Inc.) was then added to the tube and incubated at 55°C for 5 minutes. The adaptors containing p5 and p7 ends were inserted to the DNA after this step. 50 µL of stop buffer is then loaded to knock out the Tn5 enzymes. An extension mix with Bsu polymerase and nucleotides for gap fill was added. After this step, the nucleic acid library was ready and pinned inside the hydrogel bead matrix. These same steps were repeated for each of *B. subtilis* hydrogel beads and *A. hydrophila* to lyse the bacterial cells and finish library preparation for the respective hydrogel beads. As shown in FIG. 10, hydrogel beads having DNA library produced therein were seeded to a surface, and the DNA libraries released. FIG. 10, panel a) shows hydrogels with tagmented genome loaded onto a P5/P7 grafted surface. FIG. 10, panel b) shows a single stranded DNA that is eluted, seeded, and isothermally bridge amplified and stained. FIG. 10, panel c) depicts gradients of cluster density, from high density to lower density (left to right), with higher density indicating the location on the surface of where the hydrogel initially landed.

This example demonstrates preparation of hydrogel beads encapsulating a single bacterial cell inside a hydrogel bead, which can be used to process the bacterial genome and perform whole DNA library preparation inside the bead. The pore size of the hydrogel was engineered to allow the diffusion of enzymes, chemicals and smaller sized primers (< 50bps), but retained the larger sized DNA (>300bps) such that the intact whole genomic DNA and the produced DNA library was retained inside the gel microbeads during the whole process. The DNA library from the single bacterium can then be released to a specific area, for example, on flow cell surface for library seeding. Subsequently, this results in a spatial distribution of "DNA clusters" on the flow cell originating from individual bacterial cells, thus simplifying the read alignment during post processing.

### Example 5-Sequencing of Nucleic Acids from Hydrogel Beads

The following example demonstrates sequencing of DNA library from hydrogel beads encapsulating genetic material.

The DNA library prepared from Example 4 was released from the hydrogel and deposited on a surface. Fragmented DNA was eluted out of the hydrogels. Control sample was saved without heat treatment. Both samples were enriched by 12-cycle PCR reaction and assayed on a Bioanalyzer to determine the fragment distribution. After double Ampure cleanup, the *E.coli* fragments were sequenced on the MiSeq platform (Illumina, Inc., San Diego, CA). As shown in FIG. 11A, DNA fragments that eluted out of hydrogel had a fragment size distribution with an average size of around 350 bp. In contrast, sample shown in Figure 11B that was not heat treated did not show any fragments even after 12-cycle PCR reaction, indicating that the fragments were not released from the hydrogel beads. The fragments were sequenced, and 98% of the sequences aligned to enterbacteriaceae, and 39% of those aligned to *E. coli.*

### Example 6-Spatial Indexing

The following example demonstrates spatial indexing of DNA libraries prepared within hydrogel beads.

In order to demonstrate the spatial separation between different bacteria types, *E. coli, B. subtilis,* and *A. hydrophila* were chosen as a model organism. Each microorganism was embedded in its own group of hydrogel beads. Each bead type containing a single type of microorganism was processed separately through lysis treatment, and each species had its own unique index added during the library preparation protocol. The three different hydrogel beads that carried the DNA library from *E. coli, B. subtilis,* and *A. hydrophila* were then mixed to the same tube. The hydrogel beads were spun down and supernatant removed. 100 µL of mineral oil with surfactant (4.5% Span 80, 0.4% Tween 20 and 0.05% Triton X-100) was added to the beads, and vortexed for 30 seconds to resuspend the beads in the oil. 25 µL of the oil re-emulsion beads solution was then loaded to a MiSeq Flow cell. The temperature of MiSeq Flow cell was raised to 90°C for 3 minutes to denature the DNA library and the denatured library was diffused out of the hydrogel beads, followed by 60°C 6 minutes, 40°C for 2 minutes, and 20°C for 2 minutes to allow single stranded DNA library to hybridize to the surface P5/P7 primers for cluster amplification. The seeded library was clustered, linearized, and 1st base sequenced, with the results shown in FIG. 13. As shown in FIG. 13, the DNA library within the hydrogel beads were able to diffuse out of the beads and bind to the MiSeq Flow cell, where amplification occurred on the flow cell in distinct clusters for each organism.

The MiSeq flow cell seeded with library generated in hydrogel beads with these three different microorganisms was sequenced further on MiSeq (2 × 150 cycle run). Sequencing analysis, as shown in FIGs. 14A-14B, show that the run completed with a reasonable quality. Each of the three different species was detected. Read length generated from hydrogel released libraries had a very similar size range, which demonstrates that the size of the library fragments that was released from the hydrogel can be controlled by the hydrogel composition and DNA releasing protocol. A PhiX control library was also spiked in to check run quality. FIGs. 15A-15C show the MiSeq sequencing result of flow cell seeding with hydrogel beads containing *E. coli, B. subtilis,* or *A. Hydrophila* bacteria, including the raw data from MiSeq image (FIG. 15A), the clusters that aligned to specific genome (FIG. 15C), and a summary table of the pass filter of three different bacterial species (FIG. 15C).

The embodiments, examples, and figures described herein provide compositions, methods, and systems for retaining genetic material in physically confined space during the process from lysis to library generation. Some embodiments provide libraries originated from single long DNA molecule or a single cell to be released on a surface of a flow cell in confined space. Once the library from a single DNA molecule or single cell in the individual compartments are released to the surface of the flow cell, the library from each compartment gets seeded at close proximity to each other.

The term "comprising" as used herein is synonymous with "including," "containing," or "characterized by," and is inclusive or open-ended and does not exclude additional, unrecited elements or method steps.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope and spirit of the invention.

All references cited herein, including but not limited to published and unpublished applications, patents, and literature references, are incorporated herein by reference in their entirety and are hereby made a part of this specification. To the extent publications and patents or patent applications incorporated by reference contradict the disclosure contained in the specification, the specification is intended to supersede and/or take precedence over any such contradictory material.

The present application is a divisional application based on an earlier European Application No 18755643.6, which was in turn derived from PCT Application No PCT/US2018/044855. The following numbered clauses, which correspond to the claims of that earlier PCT Application as filed, form part of the present disclosure and in particular form further aspects of the invention, whether or not they appear in the present claims.

CLAUSES:
1. A bead for performing nucleic acid reactions, comprising:
   a hydrogel polymer; and
   genetic material disposed within the hydrogel, wherein the bead comprises pores that allow diffusion of a reagent through the bead while retaining the genetic material.
2. The bead of Clause 1, wherein the bead has a diameter of about 2 µm to about 120 µm.
3. The bead of any one of Clauses 1-2, wherein the hydrogel polymer comprises polyethylene glycol (PEG)-thiol, PEG-acrylate, acrylamide, N,N'-bis(acryloyl)cystamine, PEG, polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, collagen, bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations or mixtures thereof.
4. The bead of Clause 3, wherein the hydrogel polymer comprises PEG-thiol/PEG-acrylate, acrylamide/N,N'-bis(acryloyl)cystamine (BACy), or PEG/PPO.
5. The bead of any one of Clauses 1-4, wherein the genetic material is a cell, a nucleic acid, or a microbiome.
6. The bead of Clause 5, wherein the cell is a mammalian cell or bacterial cell.
7. The bead of any one of Clauses 5-6, wherein the cell is an *Escherichia coli* cell, a *Bacillus subtilis* cell, an *Aeromonas hydrophila* cell, or a fibroblast cell.
8. The bead of Clause 5, wherein the nucleic acid is DNA or RNA of 300 base pairs or greater.
9. The bead of any one of Clauses 1-8, wherein the reagent comprises enzymes, chemicals, and primers having a size of less than 50 base pairs.
10. The bead of any one of Clauses 1-9, wherein the reagent comprises lysozyme, proteinase K, random hexamers, polymerase (Φ29 DNA polymerase, Taq polymerase, Bsu polymerase), transposase (Tn5), primers (P5 and P7 adaptor sequences), ligase, catalyzing enzyme, deoxynucleotide triphosphates, buffers, or divalent cations.
11. A method of encapsulating genetic material within a hydrogel bead, comprising:
   mixing genetic material with a hydrogel polymer in solution;
   mixing the solution with an immiscible fluid to form a hydrogel bead encapsulating the genetic material, wherein the hydrogel bead comprises pores that allow diffusion of a reagent through the hydrogel bead while retaining the genetic material.
12. The method of Clause 11, wherein the hydrogel bead has a diameter of about 2 µm to about 120 µm.
13. The method any one of Clauses 11-12, wherein the hydrogel polymer comprises polyethylene glycol (PEG)-thiol, PEG-acrylate, acrylamide, N,N'-bis(acryloyl)cystamine (BACy), PEG, polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, collagen, bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations or mixtures thereof.
14. The method of Clause 13, wherein the hydrogel polymer comprises PEG-thiol/PEG-acrylate, acrylamide/N,N'-bis(acryloyl)cystamine (BACy), or PEG/PPO.
15. The method of any one of Clauses 11-14, wherein the genetic material is a cell, a nucleic acid, or a microbiome.
16. The method of Clause 15, wherein the cell is a mammalian cell or bacterial cell.
17. The method of any one of Clauses 15-16, wherein the cell is an *Escherichia coli* cell, a *Bacillus subtilis* cell, an *Aeromonas hydrophila* cell, or a fibroblast cell.
18. The method of Clause 15, wherein the nucleic acid is DNA or RNA of 300 base pairs or greater.
19. The method of any one of Clauses 11-18, wherein the reagent comprises enzymes, chemicals, and primers having a size of less than 50 base pairs.
20. The method of any one of Clauses 11-19, wherein the reagent comprises lysozyme, proteinase K, random hexamers, polymerase (Φ29 DNA polymerase, Taq polymerase, Bsu polymerase), transposase (Tn5), primers (P5 and P7 adaptor sequences), ligase, catalyzing enzyme, deoxynucleotide triphosphates, buffers, or divalent cations.
21. A method of encapsulating genetic material within a hydrogel bead, comprising:
   mixing an aqueous solution comprising a hydrogel polymer and genetic material with an immiscible fluid;
   inputting the aqueous solution into a droplet generator; and
   generating a hydrogel bead encapsulating the genetic material, wherein the hydrogel bead comprises pores that allow diffusion of a reagent through the hydrogel bead while retaining the genetic material.
22. The method of Clause 21, wherein the hydrogel bead has a diameter of about 2 µm to about 150 µm.
23. The method any one of Clauses 21-22, wherein the hydrogel polymer comprises polyethylene glycol (PEG)-thiol/PEG-acrylate, acrylamide/N,N'-bis(acryloyl)cystamine (BACy), PEG/polypropylene oxide (PPO), polyacrylic acid, poly(hydroxyethyl methacrylate) (PHEMA), poly(methyl methacrylate) (PMMA), poly(N-isopropylacrylamide) (PNIPAAm), poly(lactic acid) (PLA), poly(lactic-co-glycolic acid) (PLGA), polycaprolactone (PCL), poly(vinylsulfonic acid) (PVSA), poly(L-aspartic acid), poly(L-glutamic acid), polylysine, agar, agarose, alginate, heparin, alginate sulfate, dextran sulfate, hyaluronan, pectin, carrageenan, gelatin, chitosan, cellulose, collagen, bisacrylamide, diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, or combinations or mixtures thereof.
24. The method of Clause 23, wherein the hydrogel polymer comprises PEG-thiol/PEG-acrylate, acrylamide/N,N'-bis(acryloyl)cystamine (BACy), or PEG/PPO.
25. The method of any one of Clauses 21-24, wherein the genetic material is a cell, a nucleic acid, or a microbiome.
26. The method of Clause 25, wherein the cell is a mammalian cell or bacterial cell.
27. The method of any one of Clauses 25-26, wherein the cell is an *Escherichia coli* cell, a *Bacillus subtilis* cell, an *Aeromonas hydrophila* cell, or a fibroblast cell.
28. The method of Clause 25, wherein the nucleic acid is DNA or RNA of 300 base pairs or greater.
29. The method of any one of Clauses 21-28, wherein the reagent comprises enzymes, chemicals, and primers having a size of less than 50 base pairs.
30. The method of any one of Clauses 21-29, wherein the reagent comprises lysozyme, proteinase K, random hexamers, polymerase (Φ29 DNA polymerase, Taq polymerase, Bsu polymerase), transposase (Tn5), primers (P5 and P7 adaptor sequences), ligase, catalyzing enzyme, deoxynucleotide triphosphates, buffers, or divalent cations.
31. A method of preparing a nucleic acid library from genetic material encapsulated within a bead, comprising:
   obtaining a bead of any one of Clauses 1-10;
   amplifying genetic material encapsulated within the bead;
   performing a tagmentation reaction on the genetic material encapsulated within the bead; and
   sequencing the genetic material, thereby generating a nucleic acid library encapsulated within the bead.
32. The method of Clause 31, wherein the genetic material is a cell, and further comprising lysing the cell and extracting nucleic acids.
33. The method of Clause 32, wherein the cell is lysed with lysozyme and treated with proteinase K to extract nucleic acids.
34. The method of any one of Clauses 31-33, wherein the tagmentation reaction comprises contacting genetic material with a transposase mixture comprising adapter sequences and transposomes.
35. The method of any one of Clauses 31-34, further comprising seeding the nucleic acid library on a solid support.
36. The method of Clause 35, wherein seeding comprises degrading the bead to release the nucleic acid library from the bead.
37. The method of Clause 36, wherein the bead is degraded by contacting the bead with a cleavage mix or by heating the bead to about 90°C to release the nucleic acid library.
38. The method of Clause 37, wherein the cleavage mix comprises dithiothreitol (DTT), tris(2-carboxyethyl)phosphine (TCEP ), or tris(3-hydroxypropyl)phosphine (THP).
39. The method of any one of Clause 35-38, wherein the solid support is a flow cell device.

## Claims

1. A method of sequencing a nucleic acid from a single cell, comprising:
encapsulating the single cell within a hydrogel polymer bead;
tagmenting the nucleic acid from the single cell with a barcode and adaptor sequences within the hydrogel polymer bead, thereby generating a nucleic acid library within the hydrogel polymer bead;
seeding the hydrogel polymer bead to a surface comprising oligonucleotides immobilized thereon that bind to the adaptor sequences;
releasing the nucleic acid library from the hydrogel polymer bead, wherein the nucleic acid library binds to the surface; and
sequencing the nucleic acid library on the surface and identifying the single cell by reference to the barcode.

2. The method of claim 1, wherein the nucleic acid is DNA.

3. The method of claim 1, wherein the single cell is a mammalian cell or a bacterial cell.

4. The method of claim 1, wherein the single cell is an *Escherichia coli* cell, a *Bacillus subtilis* cell, an *Aeromonas hydrophila* cell, or a fibroblast cell.

5. The method of claim 1, wherein the hydrogel polymer bead comprises polyethylene glycol (PEG)-thiol/PEG-acrylate, acrylamide/N,N'-bis(acryloyl)cystamine (BACy), or PEG/polypropylene oxide (PPO).

6. The method of claim 1, wherein the adaptor sequences are P5 or P7 adaptor sequences.

7. The method of claim 1, wherein the oligonucleotides comprise P5 or P7 primers.

8. The method of claim 1, wherein releasing the nucleic acid library comprises elevating the temperature of the beads, contacting the beads with a reducing agent, or subjecting the beads to an electric field.

9. The method of claim 8, wherein the temperature is increased to greater than 50°C.

10. The method of claim 8, wherein the reducing agent is dithioerythritol (DTE), dithiothreitol (DTT), 2-mercaptoethanol, glutathione, thioglycolate, 2,3-dimercaptopropanol, tris(2-carboxyethyl)phosphine (TCEP), tris(hydroxymethyl)phosphine (THP), or P-[tris(hydroxymethyl)phosphine] propionic acid (THPP).

11. The method of claim 1, wherein each hydrogel polymer bead has a diameter of 2 µm to 120 µm.

12. The method of claim 1, wherein the surface is a solid support.

13. The method of claim 1, wherein the tagmentation reaction comprises contacting the nucleic acid with a transposase mixture comprising the adapter sequences and barcoded transposons.
